(19) Europäisches Patentamt
European Patent Office
Office européen des brevets

(11) Publication number: 0 270 683 B1

# (12) EUROPEAN PATENT SPECIFICATION

(45) Date of publication of patent specification: 09.09.92

(51) Int. Cl.5: C07C 275/00, C07C 275/04, C07D 213/63, C07D 237/14, C07D 239/34, C07D 241/18, A01N 47/28, A01N 47/32

(21) Application number: 87903426.2

(22) Date of filing: 26.05.87

(86) International application number: PCT/JP87/00335

(87) International publication number: WO 87/07269 (03.12.87 87/27)

## (54) ARYLOXYUREAS, PROCESS FOR THEIR PREPARATION, AND THEIR USE.

(30) Priority: 26.05.86 JP 119294/86

(43) Date of publication of application: 15.06.88 Bulletin 88/24

(45) Publication of the grant of the patent: 09.09.92 Bulletin 92/37

(84) Designated Contracting States: AT BE CH DE FR GB IT LI NL

(56) References cited:
EP-A- 0 133 918
JP-B- 415 254

TETRAHEDRON LETTERS, vol. 25, no. 48, 1984, pages 5537-5540, Pergamon Press Ltd, GB; Y. ENDO et al.: "A novel acid-catalyzed rearrangement of N-aryl-N'-aryloxyureas to biphenyl derivatives. A [5,5]-rearrangement involving three heteroatoms"

(73) Proprietor: MITSUI PETROCHEMICAL INDUSTRIES, LTD.
2-5, Kasumigaseki 3-chome Chiyoda-ku
Tokyo 100(JP)

Proprietor: KUMIAI CHEMICAL INDUSTRY CO., LTD.
4-26, Ikenohata 1-chome
Taitoh-ku Tokyo 110(JP)

(72) Inventor: HASHIMOTO, Isao
37-25, Hirata 6-chome
Iwakuni-shi Yamaguchi 741(JP)
Inventor: ISHIDA, Tatsuyoshi
2-7, Misomo 1-chome
Ohtake-shi Hiroshima 739-06(JP)
Inventor: TSURU, Kazutaka
12-2, Shouzoku-cho 5-chome
Iwakuni-shi Yamaguchi 740(JP)
Inventor: YAMADA, Yuji
3353, Kamo kikugawa-cho
Ogasa-gun Shizuoka 439(JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

Inventor: **MIYAZAWA, Takeshige**
**1809, Kamo Kikugawa-cho**
**Ogasa-gun Shizuoka 439(JP)**
Inventor: **NAKAMURA, Yasuo**
**14-15, Aobadai 1-chome**
**Kikugawa-cho, Ogasa-gun Shizuoka 439(JP)**

(74) Representative: **Wächtershäuser, Günter, Dr.**
**Tal 29**
**W-8000 München 2(DE)**

## Description

This invention relates to a novel aryloxyurea, processes for its production, and to a herbicide comprising it as an active ingredient.

Many herbicides have been used to secure quantities of crops harvested. Urea-series compounds, for example DCMU widely used as a herbicide for upland farms, have the defect that they cannot be used in paddies because of their strong phytotoxicity.

U. S. Patent Specification No. 3,332,975 discloses that m-chlorophenoxyureas which differ in chemical structure from the compounds of this invention can be used as medicines. As will be stated hereinafter, however, these compounds do not exhibit a herbicidal efficacy, and even if they do, the herbicidal efficacy is very weak.

Japanese Patent Publication No. 5254/1966 describes substituted ureas or thioureas represented by the following formula

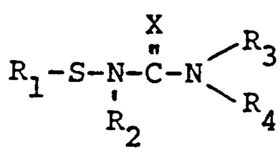

wherein $R_1$ represents a phenyl or halophenyl group, or a group of the formula

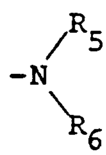

in which $R_5$ and $R_6$ are the same alkyl or aralkyl groups, or may form a piperidino or morpholino group together with the nitrogen atom to which they are bonded; $R_2$ represents a lower aliphatic hydrocarbon group, a phenyl group, or a phenyl group substituted by at least one of halogen, alkylmercapto, nitro and alkoxy; $R_3$ and $R_4$ are the same alkyl or aralkyl groups, or may form a piperidino or morpholino group together with the nitrogen atom to which they are bonded; and X is oxygen or sulfur.

It also describes that these compounds are herbicides, although it discloses no biological data.

Japanese Patent Publication No. 6999/1971 discloses that compounds of the following formula

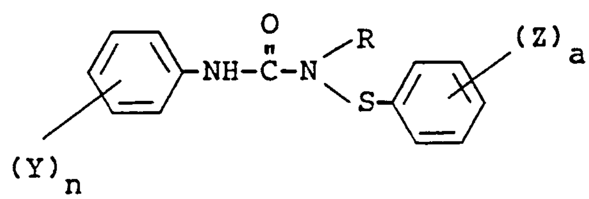

wherein R is a $C_1$-$C_4$ alkyl group; Y is F, Cl, Br or I, a $C_1$-$C_4$ alkyl group, or a $C_1$-$C_4$ alkoxy group; n is 0, or an integer of 1 to 3 when Y is the halogen atom or 1 when Y is the alkyl or alkoxy group; Z is F, Cl, Br or I or a $C_1$-$C_4$ alkyl group; and a is 0, or an integer of 1 to 5 when Z is the halogen atom, or 1 when Z is the alkyl group,

are a herbicide to be applied to plants or their growing sites.

U. S. Patent Application Serial No. 800,031 and European Patent Application EP-183174, the prior applications with respect to the present application, disclose compounds represented by the following formula (I)

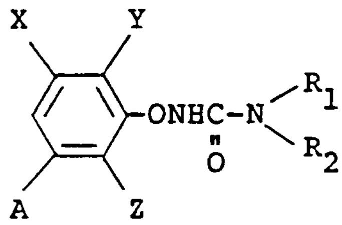

..... (I)

wherein A is a halogen atom or a trifluoromethyl group; X, Y and Z each represent a hydrogen atom, a halogen atom or a trifluoromethyl group; $R_1$ represents a $C_1$-$C_6$ alkyl group, a lower alkoxy group, a lower alkoxy-substituted lower alkyl group, a cycloalkyl-substituted lower alkyl group, a lower alkenyl group, a lower alkynyl group, a lower haloalkyl group, a lower haloalkenyl group, or a $C_3$-$C_9$ non-aromatic cyclic hydrocarbon group; $R_2$ is a hydrogen atom, a $C_1$-$C_6$ alkyl group, a lower alkenyl group or a lower alkynyl group; and $R_1$ and $R_2$, together with the nitrogen atom to which they are bonded, may form a 3- to 8-membered ring (which may be a bicyclic ring) which may contain a double bond or an oxygen atom in the ring or at least one branch.
They state that these compounds have a herbicidal efficacy.

It is an object of this invention to provide novel aryloxyureas.

Another object of this invention is to provide novel aryloxyureas which exhibit an excellent herbicidal efficacy.

Still another object of this invention is to provide novel aryloxyureas which exhibit an excellent herbicidal efficacy against weeds when applied to the weeds over a wide range of period from a preemergence stage to a growing stage.

Yet another object of this invention is to provide novel aryloxyureas which exhibit a herbicidal efficacy and yet are highly safe to crops or crop plants.

A further object of this invention is to produce the compounds of this invention having excellent herbicidal activity with industrial advantage.

Other objects and advantages of this invention will become apparent from the following description.

## DISCLOSURE OF THE INVENTION

According to this invention, the above objects and advantages of this invention are achieved firstly by an aryloxyurea represented by the following formula (I)

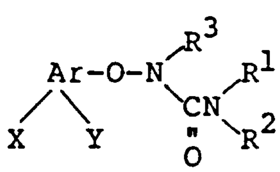

..... (I)

wherein

Ar represents a group selected from the class consisting of phenyl, pyridyl, pyridazinyl, pyrimidinyl and pyrazinyl groups,

X and Y are identical or different and each represents a hydrogen or halogen atom, or a cyano, trifluoromethyl, nitro, lower alkoxy, lower alkylthio or lower alkoxycarbonyl group,

$R^1$ represents a hydrogen atom, or a lower alkyl, lower alkenyl, lower alkynyl or $C_3$-$C_7$ cycloalkyl group,

$R^2$ represents a hydrogen atom, or a lower alkyl,

$R^1$ and $R^2$, taken together, may form a 4- to 8-membered heterocyclic group together with the nitrogen atom to which they are bonded, wherein the heterocyclic group may contain an oxygen atom as a ring-member atom or may be substituted by a lower alkyl group or a lower alkylene group,

$R^3$ represents a group of the formula $-COR^4$, a group of the formula $-CH_2OR^5$, or a lower alkyl group,

$R^4$ represents a hydrogen atom, or a lower alkyl, lower haloalkyl, lower alkoxymethyl, lower alkoxy, lower alkoxy-substituted lower alkoxy or halogen-substituted lower alkoxy group, and $R^5$ represents a hydrogen atom or a lower alkyl group; and
an acid addition salt thereof.

In formula (I), Ar is either phenyl

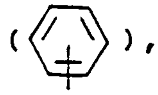

pyridyl

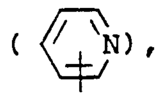

pyridazinyl

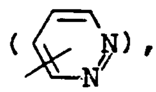

pyrimidinyl

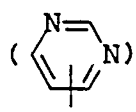

or pyrazinyl

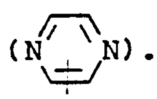

Phenyl, pyridyl and pyrazinyl are preferred as Ar, and phenyl and pyridyl are more preferred.

Pyridyl includes pyridin-2-yl and pyridin-4-yl, and pyridin-2-yl is especially preferred.

Pyridazinyl includes pyridazin-3-yl and pyridazin-4-yl.

Pyrimidinyl includes pyrimidin-2-yl and pyrimidin-4-yl. Of these, pyrimidin-4-yl is especially preferred.

Pyrazinyl means pyrazin-2-yl.

In formula (I), X and Y are atoms or groups bonded to the ring carbon atom on Ar, and each represents a hydrogen or halogen atom, or a cyano, trifluoromethyl, nitro, lower alkoxy, lower alkylthio or lower alkoxycarbonyl.

Preferred halogen atoms are fluorine, chlorine, bromine and iodine atoms.

The lower alkoxy group preferably contains a linear or branched lower alkyl moiety having 1 to 4 carbon atoms, and examples are methoxy, ethoxy, n-propoxy, iso-propoxy, n-butoxy, sec-butoxy and iso-butoxy.

The lower alkylthio group preferably contains a linear or branched alkyl moiety having 1 to 4 carbon atoms, and examples include methylthio, ethylthio, n-propylthio, iso-propylthio, n-butylthio, sec-butylthio and iso-butylthio.

The lower alkoxycarbonyl group preferably contains a linear or branched alkyl moiety having 1 to 4 carbon atoms, and examples include methoxycarbonyl, ethoxycarbonyl, n-propoxycarbonyl and n-butoxycarbonyl.

The hydrogen atom, halogen atoms, particularly chlorine and bromine atoms, trifluoromethyl and nitro are preferred as X and Y, and the hydrogen atom, chlorine atom and trifluoromethyl are especially preferred.

In accordance with the definitions of Ar, X and Y given above, examples of the group

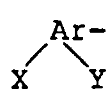

in formula (I) include substituted or unsubstituted phenyl groups such as a phenyl group, a 2-chlorophenyl group, a 3-chlorophenyl group, a 2,3-dichlorophenyl group, a 3,5-dichlorophenyl group, a 2,5-dichlorophenyl group, a 3-trifluoromethylphenyl group, a 3,5-di(trifluoromethyl)phenyl group, a 2,5-di(trifluoromethyl)phenyl group, a 2-chloro-5-trifluoromethylphenyl group, a 5-chloro-2-trifluoromethylphenyl group, a 3-chloro-5-trifluoromethylphenyl group, a 2-bromophenyl group, a 3-bromophenyl group, a 2,5-difluorophenyl group, a 2-cyanophenyl group, a 3-cyanophenyl group, a 4-cyanophenyl group, a 2-chloro-4-cyanophenyl group, a 3-chloro-2-cyanophenyl group, a 3-nitrophenyl group, a 5-chloro-2-nitrophenyl group, a 2-chloro-5-nitrophenyl group, a 2-nitro-4-trifluoromethylphenyl group, and a 6-chloro-2-nitrophenyl group; substituted or unsubstituted pyridyl groups such as a 2-pyridyl group, a 3-chloro-2-pyridyl group, a 4-chloro-2-pyridyl group, a 5-chloro-2-pyridyl group, a 6-chloro-2-pyridyl group, a 3,5-dichloro-2-pyridyl group, a 6-methoxy-2-pyridyl group, a 3-trifluoromethyl-2-pyridyl group, a 5-trifluoromethyl-2-pyridyl group, a 4-trifluoromethyl-2-pyridylgroup, a 6-trifluoromethyl-2-pyridyl group, a 3-chloro-5-trifluoromethyl-2-pyridyl group, a 5-chloro-3-trifluoromethyl-2-pyridyl group, a 6-chloro-4-trifluoromethyl-2-pyridyl group, a 3-chloro-6-trifluoromethyl-2-pyridyl group, a 5-nitro-2-pyridyl group, a 6-chloro-3-nitro-2-pyridyl group and a 3-ethoxycarbonyl-2-pyridyl group; substituted or unsubstituted pyridazinyl groups such as a 3-pyridazinyl group, a 6-chloro-3-pyridazinyl group, a 4-pyridazinyl group and a 3,6-dichloro-4-pyridazinyl group; substituted or unsubstituted pyrimidinyl groups such as a 4-pyrimidinyl group, a 6-chloro-2-methylthio-4-pyrimidinyl group and a 2-methylthio-4-pyrimidinyl group; and substituted or unsubstituted pyrazinyl groups such as a 2-pyrazinyl group and a 6-chloro-2-pyrazinyl group.

In formula (I), $R^1$ represents a hydrogen atom, or a lower alkyl, lower alkenyl, lower alkynyl or $C_3$-$C_7$ cycloalkyl group.

The lower alkyl group is preferably a linear or branched alkyl group having 1 to 4 carbon atoms, and examples are methyl, ethyl, n-propyl, iso-propyl, n-butyl, sec-butyl and tert-butyl.

The lower alkenyl group is preferably an unsaturated hydrocarbon group in which the longest carbon chain portion has 3 to 5 carbon atoms. Examples include allyl, 2-butenyl, 3-butenyl, 1-methyl-2-propenyl, 2-methyl-2-propenyl, 1,2-dimethyl-2-propenyl, 2-methyl-2-butenyl, 3-methyl-2-butenyl, and 2,3-dimethyl-2-butenyl.

The lower alkynyl group is preferably an unsaturated hydrocarbon group in which the longest carbon chain portion has 3 to 5 carbon atoms, and examples include 2-propynyl, 2-butynyl, 3-butynyl, 1-methyl-2-propynyl and 1,1-dimethyl-2-propynyl.

Examples of the $C_3$-$C_7$ cycloalkyl group are cyclopropyl, cyclobutyl, cyclopentyl, cyclohexyl and cycloheptyl.

The lower alkyl and $C_3$-$C_7$ cycloalkyl groups are especially preferred as $R^1$.

In formula (I), $R^2$ represents a hydrogen atom or a lower alkyl group.

Examples of the lower alkyl group may be the same as those given above with regard to $R^1$.

The 4- to 8-membered, preferably 6- or 7-membered, heterocyclic group which $R^1$ and $R^2$ taken together can form together with the nitrogen atom to which they are bonded, may contain an oxygen atom as a ring-member atom or may be substituted by a lower alkyl or lower alkylene group. Examples of the lower alkyl substituent may be the same as those given above with regard to $R^1$. Examples of the lower alkylene substituent are preferably alkylene groups having 2 to 4 carbon atoms, such as ethylene, trimethylene and tetramethylene. The following groups may be cited as examples of the above heterocyclic group.

<u>Four-membered groups</u>

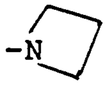 , 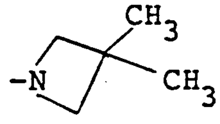 .

<u>Five-membered groups</u>

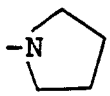 , 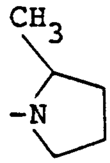 , 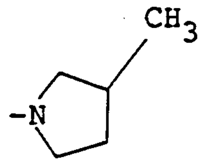 , 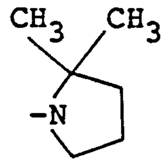 , 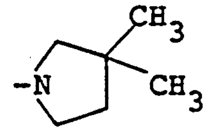 ,

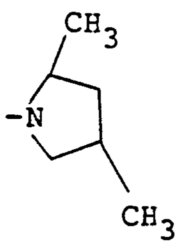 , 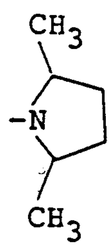 , 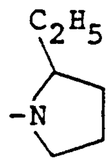 , 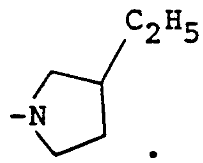 .

Six-membered groups

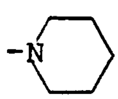 , 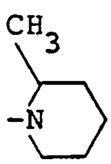 , 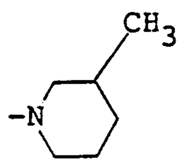 , 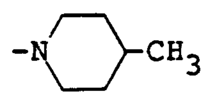 , 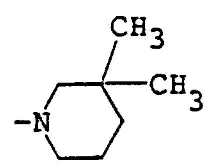 ,

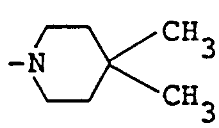 , 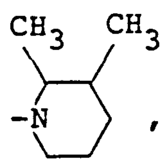 , 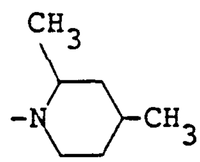 , 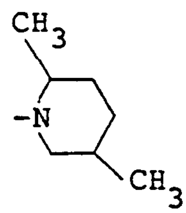

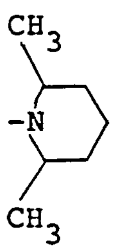 , 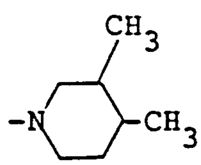 , 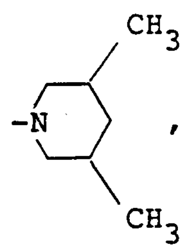 , 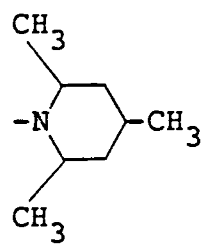 , 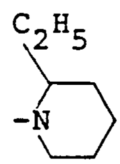 ,

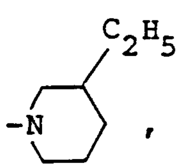 , 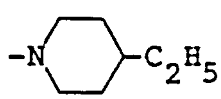 , 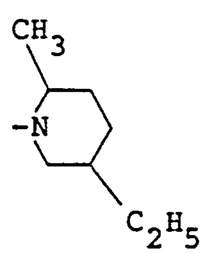 , 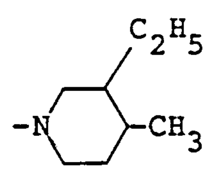

Seven-membered groups

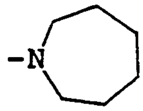, 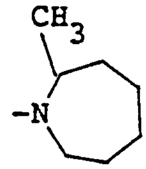, 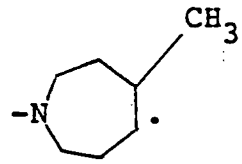.

Eight-membered groups

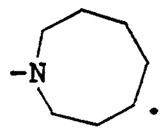.

Oxygen-containing heterocyclic groups

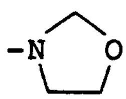, 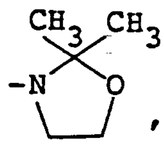, 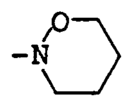, 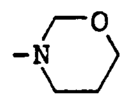, 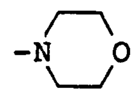,

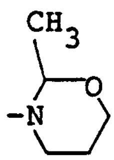, 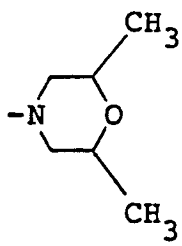.

Lower alkylene-containing heterocyclic groups (bicyclic groups)

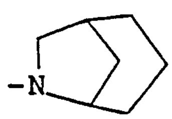, 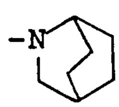, 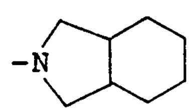, 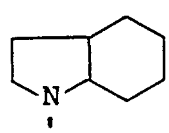,

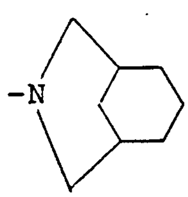, 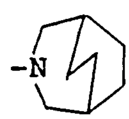, 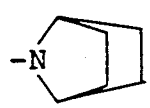, 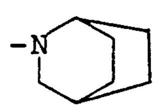.

In formula (I), $R^3$ represents a group of the formula $-COR^4$, a group of the formula $-CH_2OR^5$, or a lower alkyl group. $R^4$ represents a hydrogen atom, or a lower alkyl, lower haloalkyl, lower alkoxymethyl, lower alkoxy, lower alkoxy-substituted lower alkoxy or halogen-substituted lower alkoxy group.

Specific examples of the lower alkyl and lower alkoxy groups may be the same as those given above with regard to $R^1$ and X.

Examples of the halo in the lower haloalkyl group are fluorine, chlorine, bromine and iodine. The lower haloalkyl group is preferably a linear or branched haloalkyl group having 1 to 4 carbon atoms, such as chloromethyl, trifluoromethyl, 2-chloroethyl, 2-bromoethyl, 2,2,2-trifluoroethyl and 2,3,3,3-tetrafluoropropyl-4-chlorobutyl.

Examples of the lower alkoxy moiety of the lower alkoxymethyl group are preferably the same as those given above with regard to X. Examples of the lower alkoxymethyl group are methoxymethyl, ethoxymethyl, n-propoxymethyl and n-butoxymethyl.

Examples of the two lower alkoxy moieties in the lower alkoxy-substituted lower alkoxy group may preferably be the same as those given above with regard to X. Examples of the lower alkoxy-substituted lower alkoxy group are 2-methoxyethoxy, 2-ethoxyethoxy and 3-methoxypropoxy groups. Examples of the halogen in the halogen-substituted lower alkoxy group are fluorine, chlorine, bromine and iodine. Examples of the lower alkoxy moiety may preferably be those given above with regard to X. Examples of the halogen-substituted lower alkoxy group are 2-chloroethoxy, 2,2,2-trichloroethoxy and 2-fluoroethoxy.

From the above-given specific examples of $R^4$, the groups represented by $-COR^4$ are therefore formyl ($R^4$ = H), alkylcarbonyl ($R^4$ = lower alkyl), haloalkylcarbonyl ($R^4$ = lower haloalkyl) and alkoxymethylcarbonyl ($R^4$ = lower alkoxymethyl) and alkoxycarbonyl ($R^4$ = lower alkoxy), and specific examples of each of these groups will also be understood.

$R^5$ is a hydrogen atom or a lower alkyl group. Examples of the lower alkyl group may be the same as those given hereinabove with regard to $R^1$

The group $-CH_2OR^5$ is therefore hydroxymethyl ($R^5$ = H) or alkoxymethyl ($R^5$ = lower alkyl), and examples of the alkoxymethyl are methoxymethyl, ethoxymethyl, n-propoxymethyl and n-butoxymethyl.

Preferred compounds of formula (I) in accordance with this invention are, for example, those described in Table 1 below.

Table 1

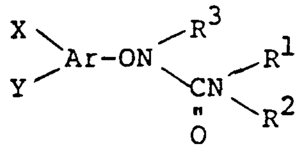

| Compound No. | 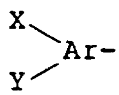 | $R^3$ | $R^1$ | $R^2$ |
|---|---|---|---|---|
| 1 | 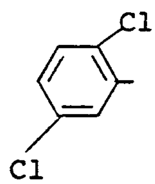 | CHO | $t\text{-}C_4H_9$ | H |
| 2 | 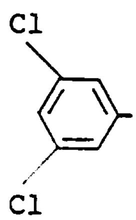 | $COCH_3$ | 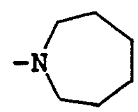 | |
| 3 | " | $CO_2CH_3$ | $i\text{-}C_3H_7$ | $CH_3$ |
| 4 | 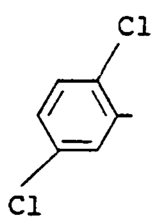 | $CH_2OH$ | $n\text{-}C_3H_7$ | H |
| 5 | 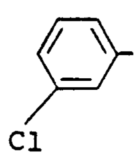 | $COCH_3$ | 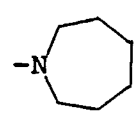 | |
| 6 | " | $COCH_2Cl$ | " | |

- to be continued -

Table 1 (continued)

| Compound No. | 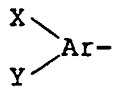 | $R^3$ | $R^1$ | $R^2$ |
|---|---|---|---|---|
| 7 | 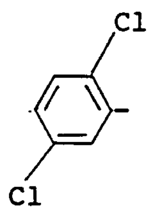 | CHO | n-$C_3H_7$ | H |
| 8 | " | $COCH_3$ | " | " |
| 9 | " | $COC_2H_5$ | " | " |
| 10 | " | $COCH_2Cl$ | " | " |
| 11 | " | $COCH_2OCH_3$ | " | " |
| 12 | " | $CO_2CH_3$ | " | " |
| 13 | " | CHO | i-$C_3H_7$ | " |
| 14 | " | $COCH_3$ | " | " |
| 15 | " | $COCH_2Cl$ | " | " |
| 16 | " | $CO_2CH_3$ | " | " |
| 17 | " | $COCH_3$ | t-$C_4H_9$ | " |
| 18 | " | $COCH_2Cl$ | " | " |
| 19 | " | $CO_2CH_3$ | " | " |
| 20 | " | $COCH_3$ | n-$C_3H_7$ | $CH_3$ |
| 21 | " | CHO | 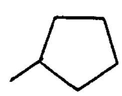 | H |

- to be continued -

Table 1 (continued)

| Compound No. | X, Y >Ar- | $R^3$ | $R^1$ | $R^2$ |
|---|---|---|---|---|
| 22 | 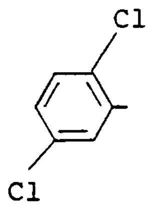 | $COCH_3$ | 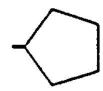 | H |
| 23 | " | $COCH_2Cl$ | " | " |
| 24 | " | $CO_2CH_3$ | " | " |
| 25 | " | $CO_2CH_3$ | 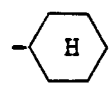 | " |
| 26 | " | $CO_2C_2H_5$ | " | " |
| 27 | " | $CO_2C_3H_7{}^n$ | " | " |
| 28 | " | $CO_2C_3H_7{}^i$ | " | " |
| 29 | 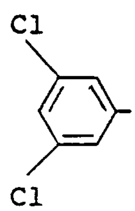 | CHO | $n-C_3H_7$ | " |
| 30 | " | $COCH_3$ | " | " |
| 31 | " | $COCH_2Cl$ | " | " |
| 32 | " | $CO_2CH_3$ | " | " |
| 33 | " | CHO | $i-C_3H_7$ | $CH_3$ |
| 34 | " | $COCH_3$ | " | " |
| 35 | " | $COC_2H_5$ | $n-C_4H_9$ | " |

- to be continued -

Table 1 (continued)

| Compound No. | 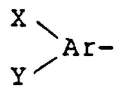 | $R^3$ | $R^1$ | $R^2$ |
|---|---|---|---|---|
| 36 | 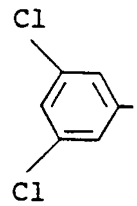 | $COCH_3$ | 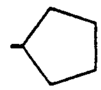 | H |
| 37 | " | $CO_2CH_3$ | " | " |
| 38 | " | $COC_2H_5$ | 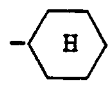 | $CH_3$ |
| 39 | " | CHO | 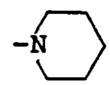 | |
| 40 | " | $COCH_3$ | " | |
| 41 | " | $COC_2H_5$ | " | |
| 42 | " | $COCH_2Cl$ | " | |
| 43 | " | $COCH_3$ | 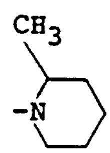 | |
| 44 | " | $COC_2H_5$ | " | |
| 45 | " | $CO_2CH_3$ | " | |
| 46 | " | CHO | 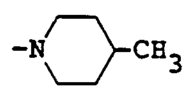 | |

- to be continued -

Table 1 (continued)

| Compound No. | 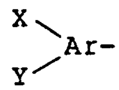 | $R^3$ | $R^1$ | $R^2$ |
|---|---|---|---|---|
| 47 | 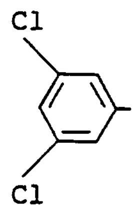 | $COCH_3$ | 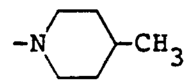 | |
| 48 | " | $COC_2H_5$ | " | |
| 49 | " | CHO | 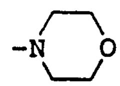 | |
| 50 | " | $COCH_3$ | " | |
| 51 | " | $COCH_2Cl$ | " | |
| 52 | " | $CO_2CH_3$ | " | |
| 53 | " | CHO | 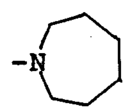 | |
| 54 | " | $COC_2H_5$ | " | |
| 55 | " | $COC_3H_7{}^n$ | " | |
| 56 | " | $COC_3H_7{}^i$ | " | |
| 57 | " | $COCH_2Cl$ | " | |
| 58 | " | $CO_2CH_3$ | " | |
| 59 | " | $COCH_3$ | 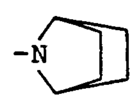 | |

- to be continued -

Table 1 (continued)

| Compound No. | X, Y >Ar- | $R^3$ | $R^1$ | $R^2$ |
|---|---|---|---|---|
| 60 | 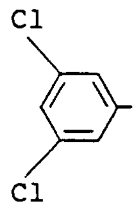 | $COCH_3$ | 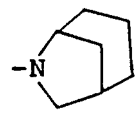 | |
| 61 | " | " | 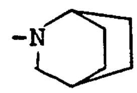 | |
| 62 | 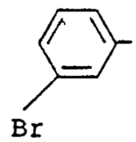 | CHO | 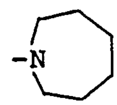 | |
| 63 | 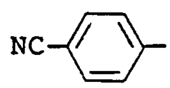 | " | " | |
| 64 | " | $COCH_3$ | " | |
| 65 | 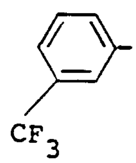 | CHO | 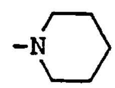 | |
| 66 | " | $COCH_3$ | " | |
| 67 | " | CHO | 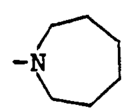 | |
| 68 | " | $COCH_3$ | " | |

- to be continued -

Table 1 (continued)

| Compound No. | 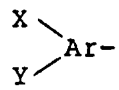 | $R^3$ | $R^1$ | $R^2$ |
|---|---|---|---|---|
| 69 | 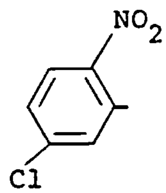 | CHO | sec-$C_4H_9$ | H |
| 70 | " | $COCH_3$ | " | " |
| 71 | " | $COCH_2Cl$ | " | " |
| 72 | " | $CO_2CH_3$ | " | " |
| 73 | " | $CH_2OH$ | " | " |
| 74 | " | $COCH_3$ | 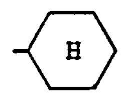 | " |
| 75 | " | $CO_2CH_3$ | " | " |
| 76 | 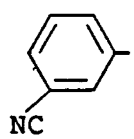 | CHO | 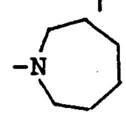 | |
| 77 | " | $COCH_3$ | " | |
| 78 | 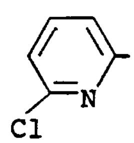 | $COCH_3$ | 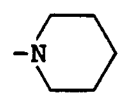 | |
| 79 | " | $COC_2H_5$ | " | |
| 80 | " | $CO_2CH_3$ | " | |
| 81 | 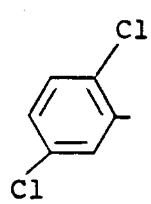 | $CH_2OC_2H_5$ | n-$C_3H_7$ | H |

- to be continued -

Table 1 (continued)

| Compound No. | X/Y>Ar- | $R^3$ | $R^1$ | $R^2$ |
|---|---|---|---|---|
| 82 | 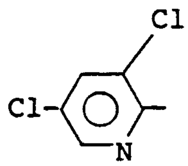 | $CO_2C_2H_5$ | 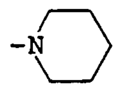 | |
| 83 | 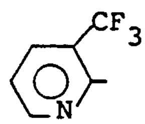 | " | " | |
| 84 | " | " | $n-C_3H_7$ | H |
| 85 | 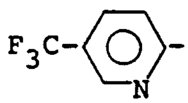 | " | " | " |
| 86 | " | " | 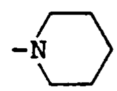 | |
| 87 | 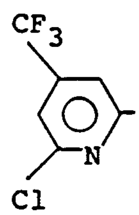 | $COCH_3$ | $n-C_3H_7$ | H |
| 88 | " | $CO_2C_2H_5$ | " | " |
| 89 | " | $CO_2C_2H_5$ | 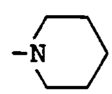 | |
| 90 | " | $CO_2CH_3$ | " | |
| 91 | 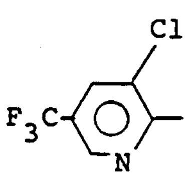 | $CO_2C_2H_5$ | $n-C_3H_7$ | H |
| 92 | " | " | 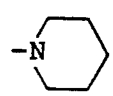 | |

- to be continued -

Table 1 (continued)

| Compound No. | X, Y >Ar- | $R^3$ | $R^1$ | $R^2$ |
|---|---|---|---|---|
| 93 | 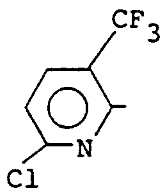 | $CO_2C_2H_5$ | 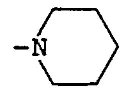 | |
| 94 | 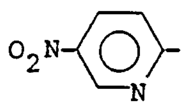 | " | " | |
| 95 | 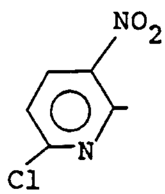 | " | n-$C_3H_7$ | H |
| 96 | 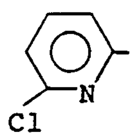 | $CO_2CH_3$ | 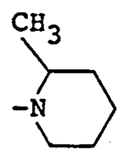 | |
| 97 | " | " | 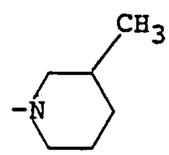 | |
| 98 | " | " | 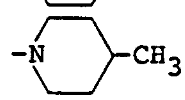 | |
| 99 | " | " | 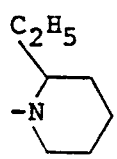 | |
| 100 | " | " | 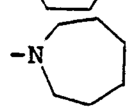 | |
| 101 | " | " | 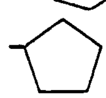 | $CH_3$ |

- to be continued -

Table 1 (continued)

| Compound No. | 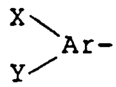 | $R^3$ | $R^1$ | $R^2$ |
|---|---|---|---|---|
| 102 | 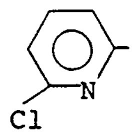 | $CO_2C_2H_5$ | 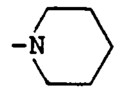 | |
| 103 | " | " | 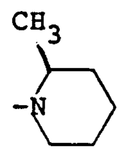 | |
| 104 | " | " | 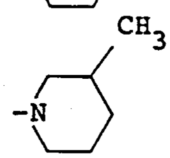 | |
| 105 | " | " | 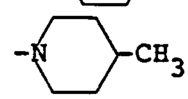 | |
| 106 | " | " | 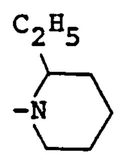 | |
| 107 | " | " | 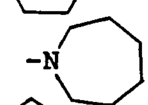 | |
| 108 | " | " | 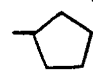 | $CH_3$ |
| 109 | 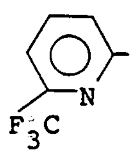 | $CO_2CH_3$ | 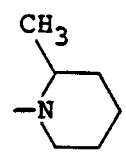 | |
| 110 | " | $CO_2C_2H_5$ | " | |
| 111 | " | $CO_2(CH_2)_2CH_3$ | " | |
| 112 | " | 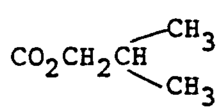 | " | |

- to be continued -

Table 1 (continued)

| Compound No. | 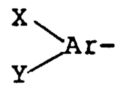 | $R^3$ | $R^1$ | $R^2$ |
|---|---|---|---|---|
| 113 | 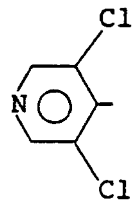 | $CO_2C_2H_5$ | $n-C_3H_7$ | H |
| 114 | 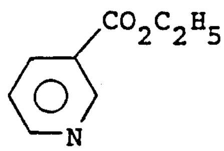 | $CO_2C_2H_5$ | 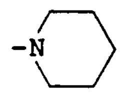 | |
| 115 | 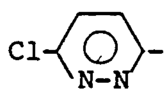 | " | $n-C_3H_7$ | H |
| 116 | " | " | 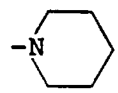 | |
| 117 | 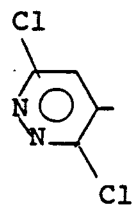 | " | " | |
| 118 | 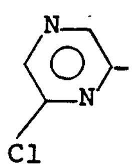 | $CO_2C_2H_5$ | $n-C_3H_7$ | H |
| 119 | " | " | 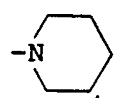 | |
| 120 | 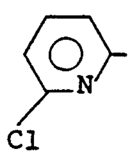 | $CO_2CH_3$ | $n-C_4H_9$ | $CH_3$ |
| 121 | " | " | $sec-C_4H_9$ | " |
| 122 | " | " | 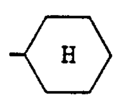 | " |

- to be continued -

Table 1 (continued)

| Compound No. | X, Y >Ar- | $R^3$ | $R^1$ | $R^2$ |
|---|---|---|---|---|
| 123 | 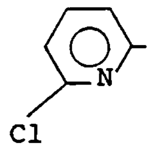 | $CO_2CH_3$ | $C_2H_5$ | $C_2H_5$ |
| 124 | " | $CO_2C_2H_5$ | n-$C_4H_9$ | $CH_3$ |
| 125 | " | " | sec-$C_4H_9$ | " |
| 126 | " | " | 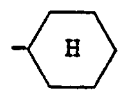 | " |
| 127 | " | " | $C_2H_5$ | $C_2H_5$ |
| 128 | " | " | 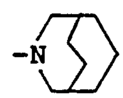 | |
| 129 | 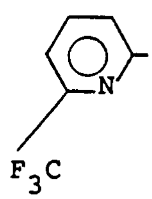 | $CO_2(CH_2)_2OCH_3$ | 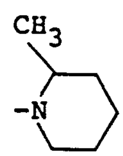 | |
| 130 | " | $CO_2CH_2CCl_3$ | " | |

- to be continued -

Table 1 (continued)

| Compound No. | X, Y >Ar- | $R^3$ | $R^1$ | $R^2$ |
|---|---|---|---|---|
| 131 | 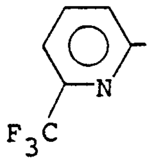 | $CO_2C_2H_5$ | 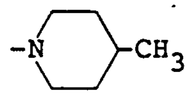 | |
| 132 | " | " | 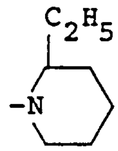 | |
| 133 | " | " | 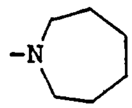 | |
| 134 | " | " | 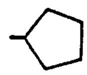 | $CH_3$ |
| 135 | " | " | 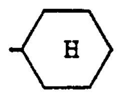 | $CH_3$ |
| 136 | " | " | n-$C_4H_9$ | $CH_3$ |
| 137 | 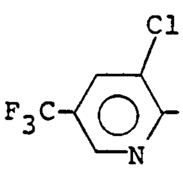 | " | 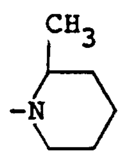 | |
| 138 | " | " | 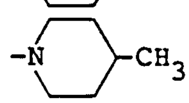 | |
| 139 | " | " | 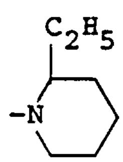 | |
| 140 | " | " | 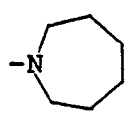 | |
| 141 | " | " | 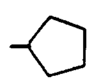 | $CH_3$ |

- to be continued -

Table 1 (continued)

| Compound No. | X, Y-Ar- | $R^3$ | $R^1$ | $R^2$ |
|---|---|---|---|---|
| 142 | 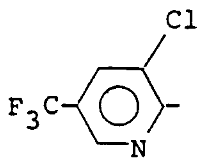 | $CO_2C_2H_5$ | sec-$C_4H_9$ | $CH_3$ |
| 143 | " | " | $C_2H_5$ | $C_2H_5$ |
| 144 | 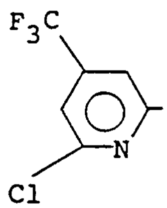 | " | 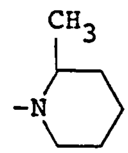 | |
| 145 | " | " | 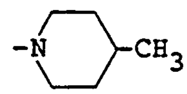 | |
| 146 | " | " | 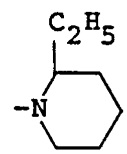 | |
| 147 | " | " | 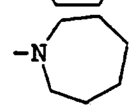 | |
| 148 | " | " | 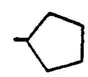 | $CH_3$ |
| 149 | " | " | sec-$C_4H_9$ | $CH_3$ |
| 150 | " | " | $C_2H_5$ | $C_2H_5$ |
| 151 | 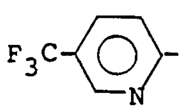 | " | 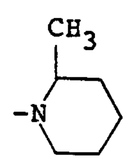 | |

- to be continued -

Table 1 (continued)

| Compound No. | 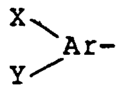 | $R^3$ | $R^1$ | $R^2$ |
|---|---|---|---|---|
| 152 | 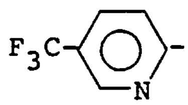 | $CO_2C_2H_5$ | 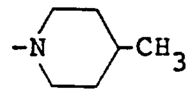 | |
| 153 | " | " | 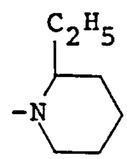 | |
| 154 | " | " | 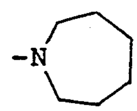 | |
| 155 | " | " | 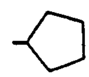 | $CH_3$ |
| 156 | " | " | sec-$C_4H_9$ | $CH_3$ |
| 157 | " | " | $C_2H_5$ | $C_2H_5$ |
| 158 | 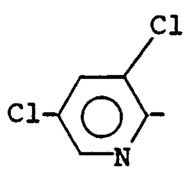 | | 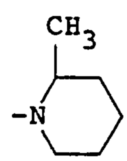 | |
| 159 | " | " | 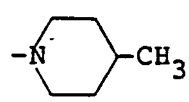 | |
| 160 | " | " | 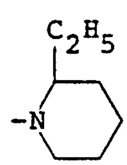 | |

- to be continued -

Table 1 (continued)

| Compound No. | 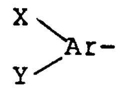 | $R^3$ | $R^1$ | $R^2$ |
|---|---|---|---|---|
| 161 | 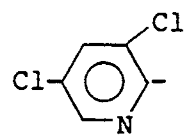 | $CO_2C_2H_5$ | 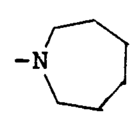 | |
| 162 | " | " | 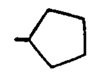 | $CH_3$ |
| 163 | " | " | sec-$C_4H_9$ | $CH_3$ |
| 164 | " | " | $C_2H_5$ | $C_2H_5$ |
| 165 | 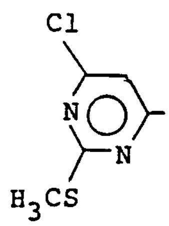 | $CH_3$ | 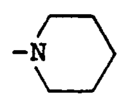 | |
| 166 | 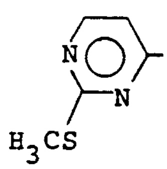 | " | " | |

Among the compounds given in Table 1, compounds Nos. 1-9, 12-19, 21-25, 27, 33-35, 39-48, 50-62, 65-69, 72, 73, 78-80, 82, 84, 86, 88-90, 92-94, 113, 116 and 119 are preferred. Especially preferred are compounds Nos. 1-3, 5, 6, 8, 12-15, 21-25, 33, 34, 39, 40, 42, 43, 45, 53, 54, 55, 56, 58, 62, 65, 67, 68, 78, 79, 80, 82, 84, 86, 89, 90, 92, 93, 94 and 1191.

The substances in accordance with this invention may be in a free form, or in the form of a salt such as an acid addition salt. Examples of the acid that constitutes the acid addition salt may include, for example, mineral acids such as hydrochloric acid, sulfuric acid and phosphoric acid, and organic acids such as chloroacetic acid, dichloroacetic acid, trichloroacetic acid, maleic acid and citric acid.

According to this invention, aryloxyureas of the following formula (I)-1, which are encompased within formula (I),

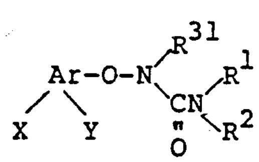

(I)-1

wherein Ar, X, Y, $R^1$ and $R^2$ are as defined with regard to formula (I), $R^{31}$ represents a group of the formula -$COR^{41}$, a group of the formula -$CH_2OR^{51}$ or a lower alkyl group, $R^{41}$ represents a lower alkyl, lower

haloalkyl, lower alkoxymethyl, lower alkoxy, lower alkoxy-substituted lower alkoxy or halogen-substituted lower alkoxy group, and $R^{51}$ represents a lower alkyl group,
can be produced by reacting a compound represented by the following formula (II)

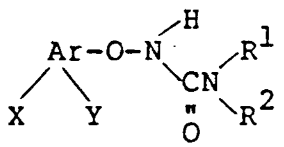 (II)

wherein Ar, X, Y, $R^1$ and $R^2$ are as defined with regard to formula (I),
with a compound represented by the following formula (III)

$R^{31}$-Cl (III)

wherein $R^{31}$ is as defined with regard to formula (I)-1,
in the presence of a base.

In formula (II), the definitions of Ar, X, Y, $R^1$ and $R^2$ are the same as those given with regard to formula (I). Accordingly, specific examples of the compound of formula (II) will be apparent to those skilled in the art from the above-given specific examples of the compound of formula (I). The compound of formula (II) can be produced by, or basically in accordance with, the methods described in European Patent Application EP-183174, Japanese Laid-Open Patent Publication No. 126065/1986 and Japanese Patent Applications Nos. 257691/1985 and 119293/1986.

As is clear from the definition of $R^{31}$, the compound represented by formula (III) is an acid chloride or chloroformate of the following formula

$R^{41}$-COCl

wherein $R^{41}$ represents a lower alkyl, lower haloalkyl, lower alkoxymethyl, lower alkoxy, lower alkoxy-substituted lower alkoxy or halogen-substituted lower alkoxy group, or
a lower alkoxymethyl chloride represented by the following formula

$R^{51}$-$OCH_2Cl$

wherein $R^{51}$ is a lower alkyl group.

In the above formula, examples of the lower alkyl group represented by $R^{41}$ and $R^{51}$ may be the same as those given above with regard to $R^1$ in formula (I). Examples of the lower haloalkyl, lower alkoxymethyl and lower alkoxy groups for $R^{41}$ may be the same as those given above with regard to $R^4$ in formula (I). Accordingly, specific examples of the acid chloride, chloroformate and lower alkoxymethyl chloride of the above formula will be apparent from the specific examples of $R^{41}$ and $R^{51}$.

The compound of formula (II) and the compound of formula (III) are reacted in the presence of a base.

The base may be an organic or an inorganic base. Examples of preferred organic bases are pyridine bases such as pyridine, picoline, lutidine and collidine and tertiary amines such as triethylamine, 1,8-diazabicyclo[5.4.0]undecene-7 and N,N-dimethylaniline. Examples of the inorganic base are $NaHCO_3$, $KHCO_3$, $Na_2CO_3$ and $K_2CO_3$.

In the reaction, the compound of formula (III) is used in an amount of 0.8 to 3 moles, preferably 1 to 2 moles, per mole of the compound of formula (II). The base is used usually in an amount of 0.5 to 10 moles, preferably 1 to 5 moles, per mole of the compound of formula (III).

The reaction temperature is usually 0 to 100 °C, preferably 0 to 60 °C, and the reaction time is usually 30 minutes to 30 hours. Preferably, the reaction is carried out with stirring.

Use of a reaction solvent is not essential. If desired, solvents inert to the reaction may be used. Examples include aromatic hydrocarbons such as benzene, toluene and xylene, halogenated hydrocarbons such as chloroform dichloromethane, carbon tetrachloride, dichloroethane, trichloroethane, tetrachloroethane, chlorobenzene and dichlorobenzene, tetrahydrofuran, ethyl acetate and dimethylformamide.

After the reaction, the desired compound can be obtained from the reaction mixture by customary methods such as those shown in Examples given hereinafter.

According to this invention, aryloxyureas of the following formula (I)-2, which correspond to formula (I) in which $R^3$ is -COH,

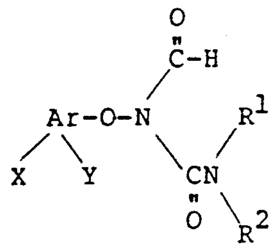

(I)-2

wherein Ar, X, Y, $R^1$ and $R^2$ are as defined with regard to formula (I),
can be produced by reacting the compound of formula (II) given above with a compound represented by the following formula (IV)

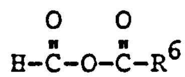

(IV)

wherein $R^6$ represents a lower alkyl group.

In formula (VI), $R^6$ represents a lower alkyl group, and its examples are the same as those shown with regard to $R^1$ in formula (I).

The compound of formula (IV) can be synthesized, for example, by a customary method of stirring formic acid and an acid anhydride represented by the general formula $(R^6CO)_2O$ at a temperature of 50 to 80 °C. The synthesized reaction mixture may, as required, be directly used in the above process of this invention without isolation of the compound (IV).

In the reaction, the compound of formula (IV) is used in an amount of 3 to 10 moles, preferably 3 to 7 moles, per mole of the compound of formula (II).

The reaction temperature is usually 40 to 80 °C, and the reaction time is usually 1 to 10 hours. The reaction is preferably carried out with stirring.

The reaction may be carried out without a solvent or in a solvent. The same solvents as those given above which can be used in the above process of this invention using the compound of formula (III) may be used.

Furthermore, according to this invention, aryloxyureas represented by the following formula (I)-3, which correspond to formula (I) in which $R^3$ is $-CH_2OH$,

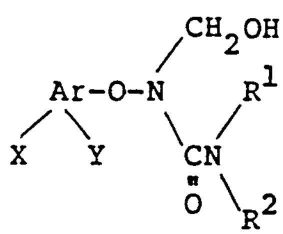

(I)-3

wherein Ar, X, Y, $R^1$ and $R^2$ are as defined with regard to formula (I),
may be produced by reacting the compound represented by formula (II) with formaldehyde.

In the reaction, 1 to 5 moles of formaldehyde is usually used per mole of the compound of formula (II).

Usually, the reaction temperature is 0 to 50 °C, and the reaction time is 0.5 to 10 hours. Desirably, the reaction is carried out with stirring.

Commercial formalin may be directly used as a source of formaldehyde in the reaction.

According to this invention, aryloxyureas represented by the following formula (I)-4, which are among the compounds of formula (I) given above,

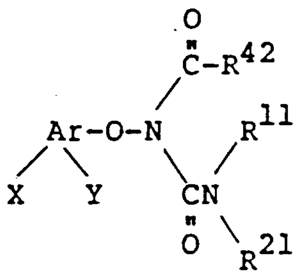

(I)-4

wherein Ar, X and Y are as defined with regard to formula (I), $R^{11}$ represents a lower alkyl, lower alkenyl, lower alkynyl or $C_3$-$C_7$ cycloalkyl group, $R^{21}$ represents a lower alkyl group, and $R^{11}$ and $R^{21}$, taken together, may form a 4- to 8-membered heterocyclic group together with the nitrogen atom to which they are bonded, said heterocyclic group optionally containing an oxygen atom as a ring-member atom or being substituted by a lower alkyl or alkylene group, and $R^{42}$ represents a lower alkoxy, lower alkoxy-substituted lower alkoxy, or halogen-substituted lower alkoxy group, can be produced by reacting a compound represented by the following formula (V)

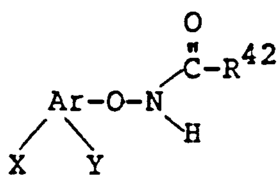

(V)

wherein Ar, X and Y are as defined with regard to formula (I), and $R^{42}$ is as defined with regard to formula (I)-4,
with a carbamoyl chloride represented by the following formula (VI)

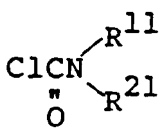

(VI)

wherein $R^{11}$ and $R^{12}$ are as defined with regard to formula (I)-4,
in the presence of a base.

The base may be an organic or an inorganic base. Examples of preferred organic bases are pyridine bases such as pyridine, picoline, lutidine and collidine and tertiary amines such as triethylamine, 1,8-diazabicyclo[5.4.0]undecene-7 and N,N-dimethylaniline. Examples of the inorganic base are $NaHCO_3$, $KHCO_3$, $Na_2CO_3$ and $K_2CO_3$.

In the reaction, the compound of formula (VI) is used in an amount of 0.8 to 3 moles, preferably 1 to 2 moles, per mole of the compound of formula (V). The base is used usually in an amount of 0.5 to 20 moles, preferably 1 to 10 moles, per mole of the compound of formula (V). The reaction temperature is usually 0 to 100 °C, preferably 20 to 80 °C, and the reaction time is usually 1 to 100 hours. Preferably, the reaction is carried out with stirring.

Use of a reaction solvent is not essential. If desired, solvents inert to the reaction may be used. Examples include aromatic hydrocarbons such as benzene, toluene and xylene, halogenated hydrocarbons such as chloroform dichloromethane, carbon tetrachloride, dichloroethane, trichloroethane, tetrachloroethane, chlorobenzene and dichloroebenzene, tetrahydrofuran, ethyl acetate and dimethylformamide.

After the reaction, the desired compound may be obtained by conventional methods such as the one shown in the Examples to be given hereinafter.

Furthermore, according to this invention, aryloxyureas represented by the following formula (I)-5, which are within the compounds of formula (I),

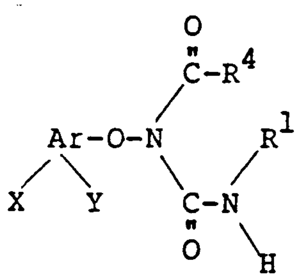

(I)-5

wherein Ar, X, Y, $R^1$ and $R^4$ are as defined hereinabove with regard to formula (I),
can be produced by reacting a compound represented by the following formula (VII)

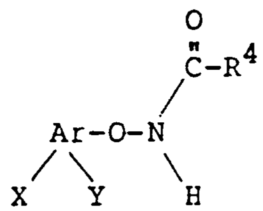

(VII)

wherein Ar, X, Y and $R^4$ are as defined above,
with an isocyanate ester represented by the following formula (VIII)

$O=C=N-R^{12}$ (VIII)

wherein $R^{12}$ represents a lower alkyl, lower alkenyl, lower alkynyl or $C_3$-$C_7$ cycloalkyl group.

In the reaction, 0.8 to 10 moles, preferably 1 to 5 moles, of the isocyanate of formula (VIII) is used per mole of the compound of formula (VII). The reaction temperature is usually 0 to 100 °C, preferably 20 to 80 °C., and the reaction time is usually 1 to 100 hours. Desirably, the reaction is carried out with stirring.

Use of a reaction solvent is not essential. If desired, solvents inert to the reaction may be used. Examples include aromatic hydrocarbons such as benzene, toluene and xylene, halogenated hydrocarbons such as chloroform, dichloromethane, carbon tetrachloride, dichloroethane, trichloroethane, tetrachloroethane, chlorobenzene, dichlorobenzene, tetrahydrofuran, ethyl acetate and dimethylformamide.

In the reaction, 0.1 to 30 mole %, based on the compound of formula (VIII) of a tertiary amine such as triethylamine may be added.

Furthermore, according to this invention, aryloxyureas of the following formula (I)-6, which are within the compounds of formula (I),

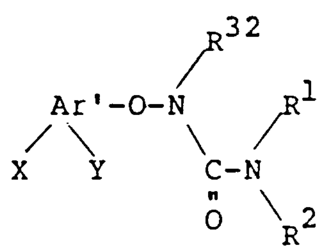

(I)-6

wherein Ar' represents a group selected from pyrimidinyl and pyridazinyl, $R^{32}$ is a lower alkyl group, and X, Y, $R^1$ and $R^2$ are as defined with regard to formula (I),
can be produced by reacting a compound represented by the following formula (IX)

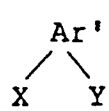

(IX)

wherein Ar' is defined above with regard to formula (I)-6, and X and Y are as defined with regard to formula (I),
with an N-hydroxyurea represented by the following formula (X)

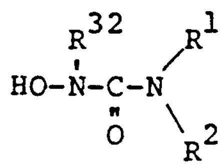

(X)

wherein $R^{32}$ is as defined with regard to formula (I)-6, and $R^1$ and $R^2$ are as defined with regard to formula (I),
in the presence of a base. The base may be a pyridine base such as pyridine, picoline, lutidine and collidine, a tertiary amine such as triethylamine, 1,8-diazabicyclo-[5,4,0]undecene-7 or N,N-dimethylaniline, or an inorganic base such as $NaHCO_3$, $KHCO_3$, $Na_2CO_3$ and $K_2CO_3$, or an alkali metal alkoxide such as sodium ethoxide or potassium t-butoxide.

The amount of the N-hydroxyurea of formula (X) is 0.5 to 10 moles, preferably 1 to 5 moles, per mole of the compound of formula (IX). The reaction temperature is usually -50 to +50 °C, preferably -40 to +40 °C, and the reaction time is usually 0.5 to 10 hours. Preferably, the reaction is carried out with stirring. Use of a reaction solvent is not essential. If desired, solvents inert to the reaction, for example an aromatic hydrocarbon such as benzene, toluene or xylene, a halogenated hydrocarbon such as chloroform, dichloromethane, carbon tetrachloride, dichloroethane, trichloroethane or benzene, tetrahydrofuran, ethyl acetate and dimethylformamide.

The aryloxyureas of formula (I) and their acid addition salts provided by this invention exhibit an excellent and characteristic herbicidal efficacy.

Accordingly, the present invention also provides a herbicide comprising the substituted aryloxyurea or its acid addition salt of the invention as a herbicidally effective ingredient.

As a herbicide, the compounds of this invention may be directly used, or as various formulations such as granules, wettable powders, emulsifiable concentrates, dusts and pulverulent agents. The compounds of this invention show better results, when used in these formulations. Herbicides in the form of these formulations can be produced from the compounds of this invention and various adjuvants, for example solid carriers such as talc, bentonite, clay, kaolin, diatomaceous earth, white carbon, vermiculite, slaked lime, silica sand, ammonium sulfate and urea, liquid carriers such as alcohols, dioxane, acetone, cyclohexanone, methylnaphthalene, dimethylformamide and dimethyl sulfoxide, emulsifiers and dispersants such as salts of alkylsulfuric esters, alkylarylsulfonate salts, polyoxyethylene glycol ethers, polyoxyethylene alkylaryl ethers and polyoxyethylene sorbitan monoalkylates, and carboxymethyl cellulose and gum arabic. As required, the proportion of the active ingredient may be adjusted. For preparation of a dust, it is suitably 0.5 to 20 % by weight, and for an emulsifiable concentrate or a wettable powder, it is suitably 5 to 70 % by weight.

The herbicide of this invention may be applied as such or in a form suitably diluted or suspended in water, etc. in an amount effective for controlling weeds. The amount of the compound of this invention used as a herbicide cannot be generalized because it varies depending upon the soil conditions, the form of the formulated preparation, the time of application, the method of application, the types of the crops or weeds to be controlled, etc. It is effective to apply it in an amount of 10 g to 5 kg per hectare.

The herbicide of this invention exhibits a better herbicidal efficacy than commercial herbicides by application to annual weeds such as barnyardgrass (Echinochloa crus-galli), umbrella plant (Cyperus difformis), nonochoria (Monochoria vaginalis), "kikashigusa" (Rotala indica), false pimpernel (Lindernia pyxidaria) and "abunome" (Dopatrium junceum) and perennial weeds such as bulrush (Scirpus juncoides), spikerush (Eleocharis acicularis), "mizugayatsuri" (Cyperus serotinus) and narrowleaf waterplaintain (Alisma canaliculatum), which occur in paddies, during their germination and growth. On the other hand, the herbicide of this invention is not phytotoxic to useful crops, particularly rice, even at high dosages, and has very high selectivity. Furthermore, by soil treatment or foliar treatment, the herbicide of this invention shows a high herbicidal efficacy against various weeds causing hazards in upland farms, for example gramineous weeds such as barnyardgrass, crabgrass (Digitaria sanguinals), green foxtail (Setaria viridis), annual bluegrass (Poa annua) and water foxtail (Alopecurus geniculatus), cyperous weeds such as rice flatsedge (Cyperus iria) and broad-leaved weeds such as redroot pigweed (Amaranthus retroflexus) and lambsquarters (Chenopodium album), and yet show high safety on principal crops such as rice, wheat, corn, soybeans

and cotton. The herbicide of this invention can also be used to control weeds in orchards, pasture land, lawns and non-agricultural lands.

If required, the herbicide of this invention may be used in combination with other agricultural chemicals and feeds, for example.

EXAMPLES

Example 1A

1-t-Butyl-3-(2,5-dichlorophenoxy)-3-formylurea (compound No. 1):-

A mixture of 1.53 g (33.3 mmoles) of formic acid and 3.24 g (31.8 mmoles) of acetic anhydride was stirred at 60 °C for 2.5 hours, and 2.20 g (7.94 mmoles) of 1-t-butyl-3-(2,5-dichlorophenoxy)urea was added. The mixture was stirred at 60 °C for 5 hours. The reaction mixture was cooled to room temperature, and 100 ml of ethyl acetate was added. The mixture was washed with a saturated aqueous solution of sodium chloride, and dried over anhydrous magnesium sulfate. The ethyl acetate was evaporated under reduced pressure. The residue was purified by silica gel column chromatography (eluting solvent: ethyl acetate/hexane) to give 1.48 g of the desired compound as colorless crystals (yield 61 %).

| | |
|---|---|
| Melting point: | 101 - 102 °C |
| Mass spectrum (FD method): | m/z 304 (molecular ion peak) |
| IR spectrum (KBr tablet method, $cm^{-1}$): | 3400, 3380, 1730, 1705, 1572, 1503, 1464, 1389, 1268, 1195, 1092, 910, 815 |

$^1$H-NMR spectrum ($CDCl_3$ solution, ppm)

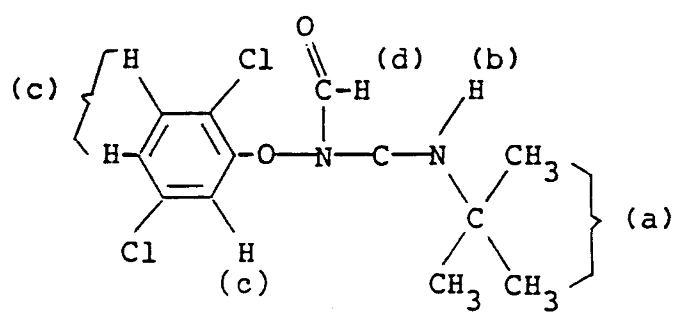

(a) 1.39 (9H,s)
(b) 6.24 (1H, br, s)
(c) 7.0 - 7.4 (3H, m)
(d) 9.22 (1H,s)

Example 2A

3-Acetyl-3-(3,5-dichlorophenoxy)-1,1-hexamethyleneurea (compound No. 2):-

In a mixed solvent of 20 ml of tetrahydrofuran and 1.6 ml of pyridine was dissolved 2.0 g (6.6 mmoles) of 3-(3,5-dichlorophenoxy)-1,1-hexamethyleneurea, and 10 ml of a tetrahydrofuran solution of 1.0 g (13.2 mmoles) of acetyl chloride was added. The mixture was stirred at 25 °C for 2 hours and then at 50 °C for 2 hours. The reaction mixture was cooled to room temperature, and 100 ml of water was added. The mixture was extracted with ethyl acetate, and the extract was washed with a dilute sulfuric acid solution and then with a saturated aqueous sodium chloride solution, and dried over anhydrous magnesium sulfate. The ethyl acetate was evaporated under reduced pressure. The residue was purified by silica gel column chromatography (eluting solvent: ethyl acetate/hexane). Recrystallization from toluene/hexane gave 1.72 g (yield 75 %) of the desired compound as colorless needle-like crystals.

| | |
|---|---|
| Melting point: | 65 - 66 °C |
| Mass spectrum (FD method): | m/z 344 (molecular ion peak) |
| IR spectrum (KBr tablet, $cm^{-1}$): | 1720, 1685, 1580, 1430, 1375, 1270, 1205, 1100 930, 845 |

$^1$H-NMR spectrum ($CDCl_3$ solution, ppm)

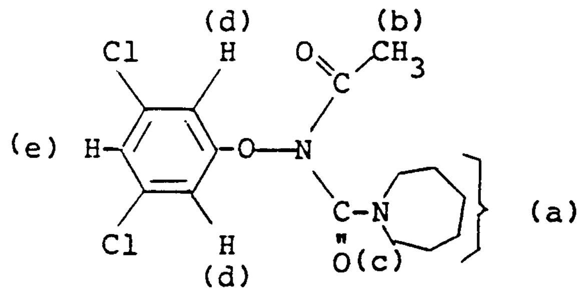

(a) 1.4 - 2.0 (8H, m)
(b) 2.27 (3H,s)
(c) 3.53 (4H, m)
(d) 7.01 (2H, d, J = 1.8Hz)
(e) 7.09 (1H, d, J = 1.8Hz)

Example 3A

3-(3,5-Dichlorophenoxy)-1-isopropyl-3-methoxy-carbonyl-1-methylurea (compound No. 3):-

In a mixed solvent of 20 ml of tetrahydrofuran and 1.9 ml of pyridine was dissolved 2.15 g (7.76 mmoles) of 3-(3,5-dichlorophenoxy)-1-isopropyl-1-methylurea, and then 10 ml of a tetrahydrofuran solution of 1.47 g (15.5 mmoles) of methyl chloroformate was added. The mixture was stirred at 25 °C for 2 hours. Water (100 ml) was added to the reaction mixture, and the mixture was extracted with ethyl acetate. The extract was washed with a saturated aqueous sodium chloride solution, and dried over anhydrous magnesium sulfate. The ethyl acetate was evaporated under reduced pressure. The residue was purified by silica gel column chromatography (eluting solvent: ethyl acetate/hexane) to give 1.14 g (yield 44 %) of the desired compound as the yellow crystals.

| | |
|---|---|
| Melting point: | 67 - 69 °C |
| Mass spectrum (FD method): | m/z 334 (molecular ion peak) |
| IR spectrum (KBr tablet, $cm^{-1}$): | 1742, 1708, 1581, 1432, 1280, 1095, 942, 840 |
| $^1$H-NMR spectrum ($CDCl_3$ solution, ppm) | |

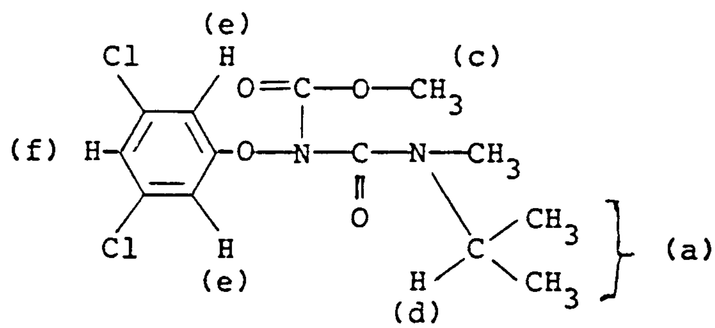

(a) 1.21 (6H, d, J = 6.6Hz)
(b) 2.91 (3H, s)
(c) 3.87 (3H, s)
(d) 4.46 (1H, m)
(e) 7.01 (2H, m)
(f) 7.07 (1H, m)

Example 4A

3-(2,5-Dichlorophenoxy)-3-hydroxymethyl-1-n-propylurea (compound No. 4):-

In 10 ml of N,N-dimethylformamide was dissolved 2.0 g (7.6 mmoles) of 3-(2,5-dichlorophenoxy)-1-n-propylurea, and then 1.87 g (22.8 mmoles) of a 37% aqueous formalin solution was added. The mixture was stirred at 25 °C for 3 hours. Water (100 ml) was added to the reaction mixture, and the mixture was extracted with ethyl acetate. The extract was washed with a saturated aqueous sodium chloride solution, and dried over anhydrous magnesium sulfate. The ethyl acetate was evaporated under reduced pressure. The residue was recrystallized from ethyl acetate and toluene to give 1.4 g (yield 63 %) of the desired compound as colorless crystals.

| | |
|---|---|
| Melting point: | 119 - 121 °C (decomp.) |
| Mass spectrum (FD method): | m/z 292 (molecular ion peak) |
| IR spectrum (KBr tablet, $cm^{-1}$): | 3320, 1665, 1580, 1530, 1470, 1275, 1215, 1085, 1045, 955, 920, 860, 800 |

$^1$H-NMR spectrum ($CDCl_3$ solution, ppm)

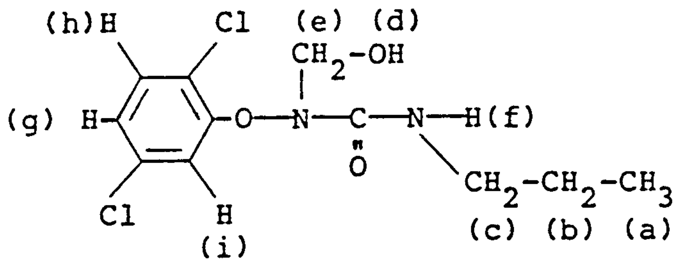

(a) 0.92 (3H,t,J = 7.2Hz)
(b) 1.3 - 1.8 (2H, m)
(c) 3.1 - 3.4 (2H, m)
(d) 3.90 (1H,t,J = 7.2Hz)
(e) 5.15 (2H,d,J = 7.2Hz)
(f) 5.8 - 6.1 (1H, m)
(g) 7.05 (1H, dd,J = 9.0, 1.8Hz)
(h) 7.35 (1H, d,J = 9.0Hz)
(i) 7.40 (1H,d,J = 1.8Hz)

Examples 5A-80A

By the same methods as in Examples 1A to 4A, compounds Nos. 5 to 80 were synthesized from the corresponding starting materials described in Table 1. The results are shown in Table 1A. A, B, C and D under the headline "method of production" correspond respectively to the methods of production described in Examples 1A, 2A, 3A and 4A.

Table 1A

| Compound No. | Method of production | Yield (%) | Melting point (°C) | IR spectrum $\nu_{C=O}$ ($cm^{-1}$) |
|---|---|---|---|---|
| 5 | B | 91 | liquid | 1715, 1690 |
| 6 | B | 51 | liquid | 1710, 1690 |
| 7 | A | 54 | 108 - 109 | 1720, 1690 |
| 8 | B | 86 | 98 - 99 | 1725, 1685 |
| 9 | B | 85 | 103 - 104 | 1725, 1685 |
| 10 | B | 88 | 135 - 137 (decomp.) | 1740, 1710 |
| 11 | B | 93 | 111 - 112 | 1740, 1700 |
| 12 | C | 84 | 97 - 98 | 1735, 1715 |
| 13 | A | 96 | liquid | 1720 (broad) |
| 14 | B | 78 | 115 - 116 | 1736, 1727 |
| 15 | B | 90 | 133 - 135 (decomp.) | 1740, 1710 |
| 16 | C | 72 | 103 - 104 | 1730 |
| 17 | B | 63 | solid solution | 1740, 1690 |
| 18 | B | 39 | 70 - 71 | 1740, 1700 |
| 19 | C | 71 | liquid | 1745, 1700 |
| 20 | B | 40 | 102 - 103 | 1705 (broad) |
| 21 | A | 75 | 84 - 85 | 1735, 1725, 1705 |
| 22 | B | 80 | 122 - 123 (decomp.) | 1735, 1725, 1685 |
| 23 | B | 85 | 130 - 132 | 1745, 1735, 1700 |

- to be continued -

Table 1A (continued)

| Compound No. | Method of production | Yield (%) | Melting point (°C) | IR spectrum $\nu_{C=O}$ ($cm^{-1}$) |
|---|---|---|---|---|
| 24 | C | 69 | 98 - 99 | 1740, 1725, 1695 |
| 25 | C | 74 | 84 - 85 | 1740, 1725, 1700 |
| 26 | C | 81 | liquid | 1740, 1710 |
| 27 | C | 97 | liquid | 1740, 1705 |
| 28 | C | 95 | liquid | 1740, 1705 |
| 29 | A | 89 | 88 - 89 | 1725, 1695 |
| 30 | B | 71 | 102 - 103 | 1730, 1690 |
| 31 | B | 75 | 86 - 87 | 1735, 1710 |
| 32 | C | 64 | 109 - 110 | 1725, 1705 |
| 33 | A | 77 | liquid | 1725, 1710, 1690 |
| 34 | B | 88 | liquid | 1710 (broad) |
| 35 | B | 79 | liquid | 1710 |
| 36 | B | 88 | 145 - 146 (decomp.) | 1725, 1685 |
| 37 | C | 83 | 131 - 132 | 1750, 1705 |
| 38 | B | 78 | 79 - 80 | 1700 (broad) |
| 39 | A | 72 | 96 - 97 | 1690 |
| 40 | B | 77 | 119 - 120 | 1725, 1700 |
| 41 | B | 95 | 89 - 90 | 1720, 1695 |
| 42 | B | 48 | 111 - 112 | 1740, 1700 |
| 43 | B | 87 | 55 - 58 | 1700 (broad) |

- to be continued -

Table 1A (continued)

| Compound No. | Method of production | Yield (%) | Melting point (°C) | IR spectrum $\nu_{C=O}$ ($cm^{-1}$) |
|---|---|---|---|---|
| 45 | C | 58 | liquid | 1740, 1700 |
| 46 | A | 51 | liquid | 1720, 1695 |
| 47 | B | 85 | 95 - 96 | 1715, 1690 |
| 48 | B | 77 | 106 - 107 | 1730, 1700 |
| 49 | A | 51 | 109 - 110 | 1720, 1690 |
| 50 | B | 94 | liquid | 1710 |
| 51 | B | 26 | solid solution | 1710 (broad) |
| 52 | C | 80 | 72 - 73 | 1745, 1710 |
| 53 | A | 36 | 114 - 115 | 1690, 1675 |
| 54 | B | 88 | 82 - 83 | 1720, 1690 |
| 55 | B | 62 | 57 - 58 | 1725, 1700, 1683 |
| 56 | B | 62 | 74 - 75 | 1705 (broad) |
| 57 | B | 36 | 76 - 77 | 1740, 1730 |
| 58 | C | 40 | liquid | 1745, 1705 |
| 59 | B | 92 | 79 - 81 | 1700 (broad) |
| 60 | B | 90 | 92 - 93 | 1700 (broad) |
| 61 | B | 95 | 72 - 75 | 1700 (broad) |
| 62 | A | 62 | 102 - 103 | 1700, 1685 |
| 63 | A | 78 | 63 - 64 | 1710, 1680 |
| 64 | B | 79 | liquid | 1690 (broad) |
| 65 | A | 87 | liquid | 1710, 1690 |

- to be continued -

Table 1A (continued)

| Compound No. | Method of production | Yield (%) | Melting point (°C) | IR spectrum $\nu_{C=O}$ ($cm^{-1}$) |
|---|---|---|---|---|
| 66 | B | 97 | liquid | 1700 (broad) |
| 67 | A | 84 | liquid | 1715, 1685 |
| 68 | B | 89 | liquid | 1690 (broad) |
| 69 | A | 67 | 78 - 79 | 1730, 1690 |
| 70 | B | 94 | 77 - 78 | 1735, 1695 |
| 71 | B | 98 | 98 - 99 | 1740, 1710 |
| 72 | C | 92 | 85 - 86 | 1745, 1705 |
| 73 | D | 91 | 70 - 72 | 1680 (broad) |
| 74 | B | 85 | 85 - 86 | 1720, 1710 |
| 75 | C | 62 | 99 - 100 | 1735, 1705 |
| 76 | A | 63 | 93 - 94 | 1685 (broad) |
| 77 | B | 66 | liquid | 1690 (broad) |
| 78 | B | 92 | 113 - 115 | 1685 (broad) |
| 79 | B | 86 | 120 - 121 | 1705, 1685 |
| 80 | C | 67 | 92 - 93 | 1740, 1700 |

Example 81A

3-(2,5-Dichlorophenoxy)-3-ethoxymethyl-1-n-propylurea (compound No. 81):-

To 2.63 g of 3-(2,5-dichlorophenoxy)-1-n-propylurea, were added, 4.14 g (20 mmoles) of anhydrous potassium carbonate and then 1.42 g (15 mmoles) of chloromethyl ethyl ether. The mixture was stirred at 50 °C for 1 hour. The reaction mixture was cooled to room temperature, and 300 ml of water was added. The mixture was extracted with ethyl acetate. The extract was washed with a saturated aqueous solution of sodium chloride and dried over anhydrous magnesium sulfate. The ethyl acetate was evaporated under reduced pressure, and the residue was purified by silica gel column chromatography (eluting solvent: ethyl acetate/hexane) to give 0.44 g (yield 14 %) of the desired compound as a pale yellow oil.

Mass spectrum (FD method): m/z 320 (molecular ion peak)

IR spectrum (neat, $cm^{-1}$):- 3430, 1643, 1575, 1465, 1398, 1375, 1235, 1160, 1079, 975, 798

$^1$H-NMR spectrum ($CDCl_3$ solution, ppm):-

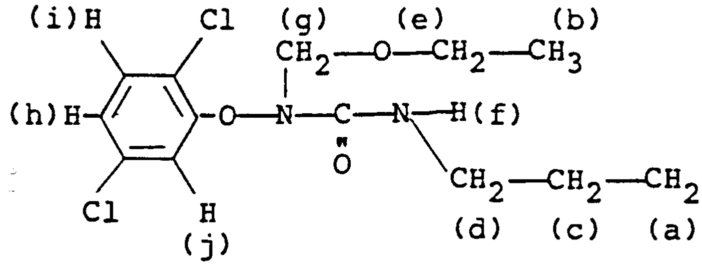

(a) 0.95 (3H,t,J = 7.2Hz)
(b) 1.29 (3H,t,J = 7.2Hz)
(c) 1.58 (2H, m)
(d) 3.21 (2H, m)
(e) 3.80 (2H,q,J = 7.2Hz)
(f) 5.1 (1H, m)
(g) 5.40 (2H, s)
(h) 6.85 (1H,dd,J = 8.8, 2.2Hz)
(i) 7.22 (1H,d,J = 8.8Hz)
(j) 7.50 (1H,d,J = 2.2Hz)

Example 82A

3-(3,5-Dichloro-2-pyridyloxy)-3-ethoxycarbonyl-1,1-pentamethyleneurea (compound No. 82):-

Ethyl N-(3,5-dichloro-2-pyridyloxy)carbamate (2.20 g; 8.76 mmoles) was dissolved in 3.5 ml of pyridine, and 1.94 g (13.1 mmoles) of 1-chloroformylpiperidine was added. The mixture was stirred at 50 °C for 20 hours. The reaction mixture was cooled to room temperature, and 250 ml of water was added. The mixture was extracted with ethyl acetate. The extract was washed with a saturated aqueous solution of sodium chloride, and dried over anhydrous magnesium sulfate. The ethyl acetate was evaporated under reduced pressure, and the residue was purified by silica gel column chromatography (eluting solvent: ethyl acetate/hexane) to give 2.75 g (yield 76 %) of the desired compound as an orange liquid.

Mass spectrum (FD method): m/z 361 (molecular ion peak)
IR spectrum (neat, $cm^{-1}$):- 1735, 1700, 1420, 1380, 1300, 1245, 1220, 1105
$^1$H-NMR spectrum($CDCl_3$ solution, ppm):-

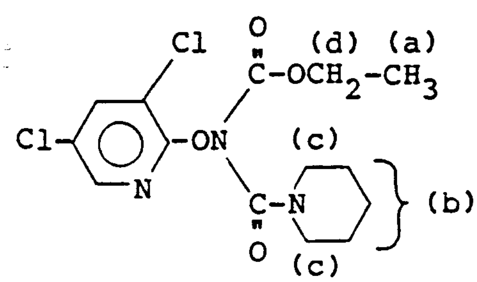

(a) 1.30 (3H, t, J = 7.0Hz)
(d) 4.29 (2H, q, J = 7.0Hz)
(e) 7.73 (1H, d, J = 2.0Hz)
(f) 8.01 (1H, d, J = 2.0Hz)

Example 83A

3-(3-Trifluoromethyl-2-pyridyloxy)-3-ethoxycarbonyl-1-n-propylurea (compound No. 83):-

Ethyl N-(3-trifluoromethyl-2-pyridyloxy)carbamate (2.30 g; 9.19 mmoles) was dissolved in 50 ml of

toluene, and 2.34 g (27.6 mmoles) of n-propyl isocyanate and 0.2 g (2.0 mmoles) of triethylamine were added, and the mixture was stirred at 45 °C for 40 hours. The toluene and the excess of n-propyl isocyanate in the reaction mixture were evaporated under reduced pressure. The residue was purified by silica gel column chromatography (eluting solvent: ethyl acetate/hexane) to give 2.61 g (yield 85 %) of the desired compound as a colorless liquid.

Mass spectrum (FD method): m/z 335 (molecular ion peak)

IR spectrum (neat, $cm^{-1}$):- 3350, 1740, 1705, 1600, 1585, 1525, 1430, 1375, 1320, 1220, 1150, 1070, 1035, 1005, 920

$^1$H-NMR spectrum ($CDCl_3$ solution, ppm):-

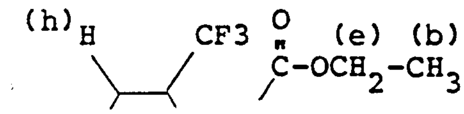

(a) 0.96 (3H, t, J = 7.0Hz)
(b) 1.20 (3H, t, J = 7.0Hz)
(c) 1.60 (2H, m)
(d) 3.30 (2H, q, J = 7.0Hz)
(e) 4.24 (2H, q, J = 7.0Hz)
(f) 7.16 (1H, dd, J = 7.4, 5.3Hz)
(g) 7.9 (1H, br.s)
(h) 7.98 (1H, d, J = 7.4Hz)
(i) 8.30 (1H, d, J = 5.3Hz)

Examples 84A-164A

The compounds Nos. 84 to 164 described in Table 1 were synthesized from the corresponding starting materials by the same methods as in Examples 82A and 83A. The results are shown in Table 2A. Methods of production E and F in Table 2A respectively correspond to the methods of production described in Examples 82A and 83A.

Table 2A

| Compound No. | Method of production | Yield (%) | Melting point (°C) | IR spectrum $\nu_{C=O}$ ($cm^{-1}$) |
|---|---|---|---|---|
| 84 | E | 41 | 74 - 76 | 1750, 1730 |
| 85 | F | 91 | liquid | 1740, 1705 |
| 86 | E | 93 | liquid | 1735, 1705 |
| 87 | F | 91 | 107 - 109 | 1715, 1685 |
| 88 | F | 95 | 70 - 72 | 1735, 1695 |
| 89 | E | 78 | liquid | 1740, 1705 |
| 90 | E | 77 | liquid | 1745, 1705 |
| 91 | F | 82 | liquid | 1740, 1710 |
| 92 | E | 97 | liquid | 1740, 1705 |
| 93 | E | 60 | liquid | 1735, 1705 |
| 94 | E | 65 | liquid | 1740, 1705 |
| 95 | F | 93 | liquid | 1740, 1705 |
| 96 | E | 36 | liquid | 1740, 1695 |
| 97 | E | 33 | liquid | 1740, 1705 |
| 98 | E | 33 | liquid | 1740, 1700 |
| 99 | E | 43 | liquid | 1740, 1695 |
| 100 | E | 30 | liquid | 1735, 1695 |
| 101 | E | 35 | liquid | 1740, 1700 |
| 102 | E | 97 | liquid | 1735, 1700 |

- to be continued -

Table 2A (continued)

| Compound No. | Method of production | Yield (%) | Melting point (°C) | IR spectrum $\nu_{C=O}$ ($cm^{-1}$) |
|---|---|---|---|---|
| 104 | E | 69 | liquid | 1730, 1705 |
| 105 | E | 72 | liquid | 1730, 1705 |
| 106 | E | 51 | liquid | 1730, 1695 |
| 107 | E | 40 | liquid | 1730, 1700 |
| 108 | E | 60 | liquid | 1730, 1700 |
| 109 | E | 66 | liquid | 1740, 1695 |
| 110 | E | 92 | liquid | 1735, 1700 |
| 111 | E | 99 | liquid | 1730, 1700 |
| 112 | E | 100 | liquid | 1730, 1700 |
| 113 | F | 74 | liquid | 1740, 1710 |
| 114 | E | 65 | liquid | 1725, 1700 |
| 115 | F | 99 | liquid | 1740, 1705 |
| 116 | E | 78 | liquid | 1740, 1700 |
| 117 | E | 26 | 125 -127 | 1760, 1705 |
| 118 | F | 99 | liquid | 1740, 1710 |
| 119 | E | 94 | liquid | 1740, 1705 |
| 120 | E | 36 | liquid | 1735, 1705 |
| 121 | E | 38 | liquid | 1740, 1695 |
| 122 | E | 32 | liquid | 1735, 1695 |

- to be continued -

Table 2A (continued)

| Compound No. | Method of production | Yield (%) | Melting point (°C) | IR spectrum $\nu_{C=O}$ ($cm^{-1}$) |
|---|---|---|---|---|
| 124 | E | 76 | liquid | 1730, 1705 |
| 125 | E | 49 | liquid | 1730, 1700 |
| 126 | E | 54 | liquid | 1730, 1695 |
| 127 | E | 53 | liquid | 1730, 1700 |
| 128 | E | 34 | liquid | 1735, 1695 |
| 129 | E | 85 | liquid | 1750, 1700 |
| 130 | E | 88 | liquid | 1750, 1705 |
| 131 | E | 82 | liquid | 1735, 1705 |
| 132 | E | 48 | liquid | 1735, 1695 |
| 133 | E | 36 | liquid | 1735, 1700 |
| 134 | E | 62 | liquid | 1735, 1700 |
| 135 | E | 46 | liquid | 1730, 1700 |
| 136 | E | 53 | liquid | 1735, 1710 |
| 137 | E | 79 | liquid | 1750, 1705 |
| 138 | E | 99 | liquid | 1740, 1705 |
| 139 | E | 72 | liquid | 1740, 1700 |
| 140 | E | 71 | liquid | 1740, 1700 |
| 141 | E | 93 | liquid | 1735, 1705 |
| 142 | E | 68 | liquid | 1740, 1705 |

- to be continued -

Table 2A (continued)

| Compound No. | Method of production | Yield (%) | Melting point (°C) | IR spectrum $\nu_{C=O}$ ($cm^{-1}$) |
|---|---|---|---|---|
| 144 | E | 82 | liquid | 1740, 1710 |
| 145 | E | 75 | 62 - 63 | 1750, 1700 |
| 146 | E | 61 | liquid | 1740, 1700 |
| 147 | E | 70 | liquid | 1740, 1700 |
| 148 | E | 76 | liquid | 1740, 1710 |
| 149 | E | 60 | liquid | 1740, 1710 |
| 150 | E | 73 | liquid | 1740, 1710 |
| 151 | E | 84 | liquid | 1730, 1700 |
| 152 | E | 85 | liquid | 1730, 1700 |
| 153 | E | 55 | liquid | 1720, 1700 |
| 154 | E | 68 | liquid | 1740, 1705 |
| 155 | E | 95 | liquid | 1730, 1705 |
| 156 | E | 72 | liquid | 1720, 1705 |
| 157 | E | 84 | liquid | 1725, 1705 |
| 158 | E | 65 | liquid | 1745, 1705 |
| 159 | E | 71 | liquid | 1740, 1705 |
| 160 | E | 55 | liquid | 1745, 1705 |
| 161 | E | 58 | liquid | 1740, 1700 |
| 162 | E | 63 | liquid | 1740, 1705 |
| 163 | E | 52 | liquid | 1740, 1710 |
| 164 | E | 30 | liquid | 1740, 1710 |

Example 165A

3-(6-Chloro-2-methylthio-4-pyrimidinyl)-1,1-pentamethyleneurea (compound No. 165):-

4.98 g (25.5 mmoles) of 4,6-dichloro-2-methylthiopyrimidine and 4.85 g (30.7 mmoles) of 3-hydroxy-3-methyl-1,1-pentamethyleneurea were dissolved in 30 ml of N,N-dimethylformamide (DMF). The solution was cooled to -30°C, and 30 ml of a DMF solution of 3.44 g (30.7 mmoles) of potassium t-butoxide was added dropwise over 15 minutes. The temperature was raised gradually from -30 °C, and after it reached +20 °C, the mixture was stirred further for 2 hours. Water (300 ml) was added to the reaction mixture, and the

mixture was extracted with ethyl acetate. The extract was washed with a saturated aqueous solution of sodium chloride, and dried over anhydrous magnesium sulfate. The ethyl acetate was evaporated under reduced pressure. The residue was purified by silica gel column chromatography (eluting solvent: ethyl acetate/hexane) to give 2.71 g (yield 34 %) of the desired compound as a colorless liquid.

| | |
|---|---|
| Mass spectrum (FD method): | m/z 316 (molecular ion peak) |
| IR spectrum (neat, $cm^{-1}$): | 1680, 1550, 1525, 1425, 1385, 1310, 1275, 1215, 1130, 1100, 990, 940. |
| $^1H$-NMR spectrum ($CDCl_3$ solution, ppm): | (a) 1.60 (6H, m), (b) 2.56 (3H, s), (c) 3.16 (3H, s), (d) 3.40 (4H, m), (e) 6.70 (1H, s). |

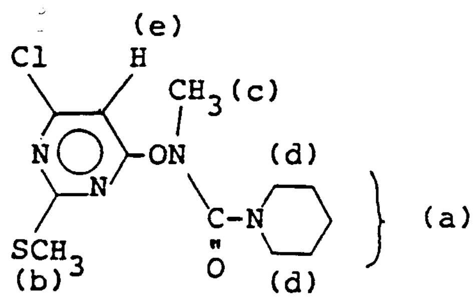

Example 166A

Compound No. 166 described in Table 1 was synthesized from the corresponding starting materials by the same method as in Example 166A.

Liquid

| | |
|---|---|
| Yield: | 83% |
| IR spectrum (neat, $cm^{-1}$): | 1675, 1562, 1545, 1410, 1345, 1225, 1130. |

Examples of formulating the herbicide of this invention will now be given. In the following Formulation Examples, all percentages are by weight.

Formulation Example 1B (granules)

The compound of this invention (10 %), 2 % of a sodium salt of lauryl sulfate, 5 % of sodium ligninsulfonate, 2 % of carboxymethyl cellulose and 81 % of clay were uniformly mixed and pulverized.

Water (20 parts) was added to 80 parts of the resulting mixture and they were kneaded. The kneaded mixture was molded into particles having a size of 14 to 32 mesh by an extrusion granulator, and then dried to form granules.

Formulation Example 2B (dust)

A sodium salt of lauryl sulfate (2 %), 5 % of sodium ligninsulfonate, 2 % of carboxymethyl cellulose and 91 % of a clay/montmorillonite mixture were uniformly mixed and pulverized. The mixture (78 parts) was kneaded with 22 parts of water, and the kneaded mixture was processed into particles having a size of 14 to 32 mesh by an extrusion granulator and then dried to form a base composition for adsorption. Twenty parts of a solution of 20 % of the compound of this invention in 80 % of polyethylene glycol was adsorbed uniformly on 80 parts of the base composition to form a dust.

Formulation Example 3B (wettable powder)

The compound of the invention (10 %), 85 % of diatomaceous earth, 2 % of sodium dinaphthylmethanedisulfonate and 3 % of sodium ligninsulfonate were uniformly mixed and pulverized to form a wettable powder.

Formulation Example 4B (emulsifiable concentrate)

The compound of this invention (30 %), 20 % of cyclohexanone, 11 % of polyoxyethylene alkylaryl

ether, 4 % of calcium alkylbenzenesulfonate and 35 % of methylnaphthalene were dissolved uniformly to form an emulsifiable concentrate.

Formulation Example 5B (dust)

The compound of this invention (4 %), 5 % of diatomaceous earth and 91 % of clay were uniformly mixed and pulverized to form a dust.

The efficacy of the herbicide of this invention will now be illustrated by the following Test Examples.

Test Example 1C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied: 4 kg per hectare as an active ingredient). The plants were then grown in a greenhouse, and 30 days after the treatment, the herbicidal activity of the compound of the invention was examined according to the standards given in Table 1C. The results are shown in Table 2C.

Table 1C

| Index | Herbicidal efficacy and phytotoxicity |
|---|---|
| 5 | Withered |
| 4.5 | Herbicidal efficacy of 90 to 99 % (phytotoxic) |
| 4 | Herbicidal efficacy of 80 to 89 % (phytotoxic) |
| 3.5 | Herbicidal efficacy of 70 to 79 % (phytotoxic) |
| 3 | Herbicidal efficacy of 60 to 69 % (phytotoxic) |
| 2.5 | Herbicidal efficacy of 50 to 59 % (phytotoxic) |
| 2 | Herbicidal efficacy of 40 to 49 % (phytotoxic) |
| 1.5 | Herbicidal efficacy of 30 to 39 % (phytotoxic) |
| 1 | Herbicidal efficacy of 20 to 29 % (phytotoxic) |
| 0.5 | Herbicidal efficacy of 19 to 1% (phytotoxic) |
| 0 | No herbicidal efficacy (no phytotoxicity) |

Table 2C

| Test compound No. | Herbicidal effect | | | |
|---|---|---|---|---|
| | barnyard-grass | umbrella plant | monochoria | bulrush |
| 1 | 5 | 5 | 5 | 5 |
| 2 | 5 | 5 | 5 | 5 |
| 3 | 5 | 5 | 5 | 5 |
| 4 | 5 | 5 | 5 | 5 |
| 5 | 5 | 5 | 5 | 5 |
| 6 | 5 | 5 | 5 | 5 |
| 7 | 5 | 5 | 5 | 5 |
| 8 | 5 | 5 | 5 | 5 |
| 9 | 5 | 4.5 | 5 | 5 |
| 10 | 5 | 5 | 5 | 5 |
| 11 | 5 | 5 | 5 | 5 |
| 12 | 5 | 5 | 5 | 5 |
| 13 | 5 | 5 | 5 | 5 |
| 14 | 5 | 5 | 5 | 5 |
| 15 | 5 | 5 | 5 | 5 |
| 16 | 5 | 5 | 5 | 5 |
| 17 | 5 | 5 | 5 | 5 |
| 18 | 5 | 5 | 5 | 5 |
| 19 | 5 | 5 | 5 | 5 |
| 20 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 21 | 5 | 5 | 5 | 5 |
| 22 | 5 | 5 | 5 | 5 |
| 23 | 5 | 5 | 5 | 5 |
| 24 | 5 | 5 | 5 | 5 |
| 25 | 5 | 5 | 5 | 5 |
| 26 | 5 | 5 | 5 | 5 |
| 27 | 5 | 5 | 5 | 5 |
| 28 | 5 | 5 | 5 | 5 |
| 29 | 5 | 5 | 5 | 5 |
| 30 | 5 | 5 | 5 | 5 |
| 31 | 5 | 5 | 5 | 5 |
| 32 | 5 | 5 | 5 | 5 |
| 33 | 5 | 5 | 5 | 5 |
| 34 | 5 | 5 | 5 | 5 |
| 35 | 5 | 5 | 5 | 5 |
| 36 | 5 | 5 | 5 | 5 |
| 37 | 5 | 5 | 5 | 5 |
| 38 | 5 | 5 | 5 | 5 |
| 39 | 5 | 5 | 5 | 5 |
| 40 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 41 | 5 | 5 | 5 | 5 |
| 42 | 5 | 5 | 5 | 5 |
| 43 | 5 | 5 | 5 | 5 |
| 44 | 5 | 5 | 5 | 5 |
| 45 | 5 | 5 | 5 | 5 |
| 46 | 5 | 5 | 5 | 5 |
| 47 | 5 | 5 | 5 | 5 |
| 48 | 5 | 5 | 5 | 5 |
| 49 | 5 | 5 | 5 | 5 |
| 50 | 5 | 5 | 5 | 5 |
| 51 | 5 | 5 | 5 | 5 |
| 52 | 5 | 5 | 5 | 5 |
| 53 | 5 | 5 | 5 | 5 |
| 54 | 5 | 5 | 5 | 5 |
| 55 | 5 | 5 | 5 | 5 |
| 56 | 5 | 5 | 5 | 5 |
| 57 | 5 | 5 | 5 | 5 |
| 58 | 5 | 5 | 5 | 5 |
| 59 | 5 | 5 | 5 | 5 |
| 60 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 61 | 5 | 5 | 5 | 5 |
| 62 | 5 | 5 | 5 | 5 |
| 63 | 5 | 5 | 5 | 5 |
| 64 | 5 | 5 | 5 | 5 |
| 65 | 5 | 5 | 5 | 5 |
| 66 | 5 | 5 | 5 | 5 |
| 67 | 5 | 5 | 5 | 5 |
| 68 | 5 | 5 | 5 | 5 |
| 69 | 5 | 5 | 5 | 5 |
| 70 | 5 | 5 | 5 | 4 |
| 71 | 5 | 5 | 5 | 5 |
| 72 | 5 | 5 | 5 | 2.5 |
| 73 | 5 | 5 | 5 | 5 |
| 74 | 5 | 5 | 5 | 5 |
| 75 | 5 | 5 | 5 | 5 |
| 76 | 5 | 5 | 5 | 5 |
| 77 | 5 | 5 | 5 | 5 |
| 78 | 5 | 5 | 5 | 5 |
| 79 | 5 | 5 | 5 | 5 |
| 80 | 5 | 5 | 5 | 5 |

- to be continued -

Table 2C (continued)

| Test compound No. | Herbicidal effect | | | |
|---|---|---|---|---|
| | barnyard-grass | umbrella plant | monochoria | bulrush |
| 81 | 5 | 5 | 5 | 5 |
| Comparison** | 0 | 0 | 0 | 0 |

*

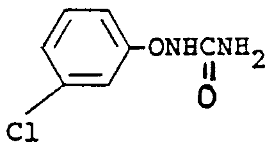

(the compound described in U.S. 3,332,975)

Test Example 2C (selective herbicidal test between rice and barnyardgrass)

Paddy soil was filled into Wagner pots (1/5,000 a), and after puddling, seeds of barnyardgrass were sown and grown to the 2-leaf stage in a greenhouse. The number of barnyardgrass plants in the two-leaf stage per pot was adjusted to 15. A predetermined amount of a wettable powder of each of the test compounds, prepared in accordance with Formulation Example 3B, was diluted with water, and dropped onto the water surface. Separately, rice seedlings in the two-leaf stage were transplanted in other Wagner pots (1/5,000 a). On the next day to the day of transplantation, the test compound was similarly dropped onto the water surface. After the treatment, the rice seedlings were grown for 30 days in a greenhouse. The herbicidal activity and phytotoxicity of the test compound were examined in accordance with the standards given in Table 1C. The results are shown in Table 3C.

Table 3C

| Test compound No. | Amount applied (kg/ha) | Herbicidal efficacy | Phytotoxicity |
|---|---|---|---|
| | | barnyardgrass in the 2-leaf stage | rice |
| 2 | 0.5<br>0.25<br>0.125 | 5<br>5<br>5 | 1<br>1<br>0 |
| 3 | 0.5<br>0.25<br>0.125 | 5<br>5<br>5 | 2<br>0<br>0 |
| 5 | 0.5<br>0.25<br>0.125 | 5<br>5<br>5 | 1<br>1<br>0 |
| 7 | 1<br>0.5<br>0.25 | 5<br>5<br>3.5 | 0<br>0<br>0 |
| 13 | 0.5<br>0.25<br>0.125 | 5<br>5<br>5 | 2<br>0<br>0 |
| 21 | 0.5<br>0.25<br>0.125 | 5<br>5<br>5 | 0<br>0<br>0 |
| 23 | 0.5<br>0.25<br>0.125 | 5<br>5<br>5 | 0<br>0<br>0 |

- to be continued -

Table 3C (continued)

| Test compound No. | Amount applied (kg/ha) | Herbicidal efficacy | Phytotoxicity |
|---|---|---|---|
| | | barnyardgrass in the 2-leaf stage | rice |
| 33 | 0.5<br>0.25<br>0.125 | 5<br>5<br>5 | 0<br>0<br>0 |
| 34 | 0.5<br>0.25<br>0.125 | 5<br>5<br>5 | 3<br>1<br>0 |
| 39 | 0.5<br>0.25<br>0.125 | 5<br>5<br>5 | 1<br>0<br>0 |
| 40 | 0.5<br>0.25<br>0.125 | 5<br>5<br>5 | 1<br>0<br>0 |
| 42 | 0.5<br>0.25<br>0.125 | 5<br>5<br>5 | 2<br>1<br>0 |
| 43 | 0.5<br>0.25<br>0.125 | 5<br>5<br>5 | 1<br>1<br>0 |
| 45 | 0.5<br>0.25<br>0.125 | 5<br>5<br>5 | 0<br>0<br>0 |

- to be continued -

Table 3C (continued)

| Test compound No. | Amount applied (kg/ha) | Herbicidal efficacy | Phytotoxicity |
|---|---|---|---|
| | | barnyardgrass in the 2-leaf stage | rice |
| 46 | 0.5<br>0.25<br>0.125 | 5<br>5<br>5 | 1<br>1<br>0 |
| 47 | 0.5<br>0.25<br>0.125 | 5<br>5<br>5 | 0<br>0<br>0 |
| 53 | 0.5<br>0.25<br>0.125 | 5<br>5<br>5 | 2<br>1<br>1 |
| 54 | 0.5<br>0.25<br>0.125 | 5<br>5<br>5 | 1<br>0<br>0 |
| 55 | 0.5<br>0.25<br>0.125 | 5<br>5<br>5 | 0<br>0<br>0 |
| 56 | 0.5<br>0.25<br>0.125 | 5<br>5<br>5 | 0<br>0<br>0 |
| 57 | 0.5<br>0.25<br>0.125 | 5<br>5<br>5 | 1<br>1<br>0 |

- to be continued -

Table 3C (continued)

| Test compound No. | Amount applied (kg/ha) | Herbicidal efficacy | Phytotoxicity |
|---|---|---|---|
| | | barnyardgrass in the 2-leaf stage | rice |
| 58 | 0.5<br>0.25<br>0.125 | 5<br>5<br>5 | 0<br>0<br>0 |
| 59 | 0.5<br>0.25<br>0.125 | 5<br>5<br>5 | 1<br>1<br>1 |
| 60 | 0.5<br>0.25<br>0.125 | 5<br>5<br>5 | 2<br>0<br>0 |
| 61 | 0.5<br>0.25<br>0.125 | 5<br>5<br>5 | 2<br>0<br>0 |
| 62 | 0.5<br>0.25<br>0.125 | 5<br>5<br>5 | 2<br>1<br>1 |
| 65 | 0.5<br>0.25<br>0.125 | 5<br>5<br>5 | 3<br>2<br>0 |
| 67 | 0.5<br>0.25<br>0.125 | 5<br>5<br>5 | 3<br>3<br>1 |

- to be continued -

Table 3C (continued)

| Test compound No. | Amount applied (kg/ha) | Herbicidal efficacy | Phytotoxicity |
|---|---|---|---|
| | | barnyardgrass in the 2-leaf stage | rice |
| 68 | 0.5<br>0.25<br>0.125 | 5<br>5<br>5 | 3<br>1<br>1 |
| 76 | 0.5<br>0.25<br>0.125 | 5<br>5<br>2 | 1<br>1<br>0 |

Test Example 3C (herbicidal test by soil treatment in an upland farm)

Upland farm soil was filled in plastic pots (120 $cm^2$), and seeds of barnyardgrass, crabgrass, redroot pigweed and rice flatsedge were sown and covered with the soil. Each of the test compounds was formulated into a wettable powder in accordance with Formulation Example 3B. A predetermined amount of the wettable powder was diluted with water, and using a small-sized sprayer, was uniformly sprayed onto the soil surface at a rate of 1000 liters/hectare (the amount applied: 4 kg per hectare as the active ingredient). The plants were grown for 20 days in a greenhouse after the treatment, and the herbicidal efficacy and phytotoxicity were examined in accordance with the standards given in Table 1C. The results are shown in Table 4C.

Table 4C

| Test compound No. | Herbicidal efficacy | | | |
|---|---|---|---|---|
| | barnyard-grass | crab-grass | redroot pigweed | rice flatsedge |
| 1 | 5 | 5 | 5 | 5 |
| 2 | 5 | 5 | 5 | 5 |
| 3 | 5 | 5 | 5 | 5 |
| 4 | 5 | 5 | 5 | 5 |
| 5 | 5 | 5 | 5 | 5 |
| 6 | 5 | 5 | 5 | 5 |
| 7 | 5 | 5 | 5 | 5 |
| 8 | 5 | 5 | 5 | 5 |
| 9 | 5 | 5 | 5 | 5 |
| 11 | 5 | 4 | 5 | 5 |
| 12 | 5 | 5 | 5 | 5 |
| 13 | 5 | 5 | 5 | 5 |
| 14 | 5 | 5 | 5 | 5 |
| 15 | 5 | 5 | 5 | 5 |
| 16 | 5 | 5 | 5 | 5 |
| 17 | 5 | 5 | 5 | 5 |
| 18 | 5 | 5 | 5 | 5 |

- to be continued -

Table 4C (continued)

| Test compound No. | Herbicidal efficacy | | | |
|---|---|---|---|---|
| | barnyard-grass | crab-grass | redroot pigweed | rice flatsedge |
| 19 | 5 | 5 | 5 | 5 |
| 20 | 5 | 4 | 2 | 5 |
| 21 | 5 | 5 | 5 | 5 |
| 22 | 5 | 5 | 5 | 5 |
| 23 | 5 | 5 | 1.5 | 5 |
| 24 | 5 | 5 | 5 | 5 |
| 25 | 5 | 5 | 3 | 5 |
| 26 | 5 | 5 | 0 | 5 |
| 27 | 5 | 4 | 0 | 5 |
| 29 | 5 | 5 | 4 | 5 |
| 30 | 5 | 5 | 5 | 5 |
| 31 | 5 | 5 | 2 | 5 |
| 32 | 4 | 5 | 4 | 5 |
| 33 | 5 | 5 | 5 | 5 |
| 34 | 5 | 5 | 5 | 5 |
| 36 | 3 | 5 | 0 | 5 |
| 37 | 5 | 5 | 1 | 5 |

- to be continued -

Table 4C (continued)

| Test compound No. | Herbicidal efficacy | | | |
|---|---|---|---|---|
| | barnyard-grass | crab-grass | redroot pigweed | rice flatsedge |
| 39 | 5 | 5 | 4 | 5 |
| 40 | 5 | 5 | 4 | 5 |
| 42 | 5 | 5 | 5 | 5 |
| 43 | 5 | 5 | 5 | 5 |
| 45 | 5 | 5 | 2 | 5 |
| 46 | 5 | 5 | 5 | 5 |
| 47 | 5 | 5 | 5 | 5 |
| 49 | 5 | 5 | 5 | 5 |
| 50 | 5 | 5 | 2 | 5 |
| 51 | 5 | 3 | 1 | 5 |
| 52 | 5 | 5 | 2 | 5 |
| 53 | 5 | 5 | 5 | 5 |
| 54 | 5 | 5 | 5 | 5 |
| 55 | 5 | 5 | 5 | 5 |
| 56 | 5 | 5 | 3 | 5 |
| 57 | 5 | 5 | 5 | 5 |
| 58 | 5 | 5 | 5 | 5 |

- to be continued -

Table 4C (continued)

| Test compound No. | Herbicidal efficacy | | | |
|---|---|---|---|---|
| | barnyard-grass | crab-grass | redroot pigweed | rice flatsedge |
| 59 | 5 | 5 | 5 | 5 |
| 60 | 5 | 5 | 5 | 5 |
| 61 | 5 | 5 | 5 | 5 |
| 62 | 5 | 5 | 5 | 5 |
| 63 | 5 | 5 | 5 | 5 |
| 64 | 5 | 5 | 5 | 5 |
| 65 | 5 | 5 | 5 | 5 |
| 66 | 5 | 5 | 5 | 5 |
| 67 | 5 | 5 | 5 | 5 |
| 68 | 5 | 5 | 5 | 5 |
| 69 | 5 | 5 | 1 | 5 |
| 70 | 5 | 5 | 3 | 5 |
| 71 | 5 | 5 | 0 | 5 |
| 72 | 5 | 5 | 0 | 5 |
| 73 | 5 | 5 | 0 | 5 |
| 74 | 2 | 5 | 0 | 5 |

- to be continued -

Table 4C (continued)

| Test compound No. | Herbicidal efficacy | | | |
|---|---|---|---|---|
| | barnyard-grass | crab-grass | redroot pigweed | rice flatsedge |
| 75 | 5 | 3 | 0 | 5 |
| 76 | 5 | 5 | 5 | 5 |
| 77 | 5 | 5 | 5 | 5 |
| 81 | 5 | 5 | 3 | 5 |

Test Example 4C (herbicidal efficacy and phytotoxicity by soil treatment in an upland farm

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar-beet were sown and covered with the soil. Each of the test compounds was formulated into a wettable powder in accordance with Formulation Example 3B. A predetermined amount of the wettable powder was diluted with water and uniformly sprayed onto the soil surface by a small-sized sprayer at a rate of 1000 liters per hectare. The plants were grown in a greenhouse for 30 days after the treatment. The herbicidal efficacy and phytotoxicity were examined in accordance with the standards given in Table 1C. The results are shown in Table 5C.

Table 5C

| Test compound No. | Amount applied (kg/ha) | Herbicidal efficacy: barnyard-grass | Herbicidal efficacy: crab-grass | Herbicidal efficacy: green foxtail | Herbicidal efficacy: redroot pigweed | Herbicidal efficacy: rice flatsedge | Phytotoxicity: rice | Phytotoxicity: wheat | Phytotoxicity: corn | Phytotoxicity: soy beans | Phytotoxicity: cotton | Phytotoxicity: sugar-beet |
|---|---|---|---|---|---|---|---|---|---|---|---|---|
| 2 | 2 | 5 | 5 | 5 | 5 | 5 | 0.5 | 1.5 | 3.5 | 0 | 0 | 1 |
| | 1 | 5 | 5 | 5 | 5 | 5 | 0 | 0.5 | 2.5 | 0 | 0 | 0.5 |
| | 0.5 | 4.5 | 5 | 5 | 5 | 5 | 0 | 0 | 2.5 | 0 | 0 | 0.5 |
| 3 | 2 | 5 | 5 | 5 | 5 | 5 | 1 | 3 | 5 | 0 | 0 | 2 |
| | 1 | 5 | 5 | 5 | 5 | 5 | 1 | 1 | 4 | 0 | 0 | 2 |
| | 0.5 | 5 | 5 | 1 | 5 | 5 | 0 | 0 | 2 | 0 | 0 | 2 |
| 5 | 2 | 5 | 5 | | 5 | 5 | 2 | 1 | 4 | 1 | | 1 |
| | 1 | 5 | 5 | | 5 | 5 | 1 | 1 | 2 | 0 | 1 | 0 |
| | 0.5 | 5 | 5 | | 5 | 5 | 0 | 1 | 1 | 0 | | 0 |
| 6 | 2 | 5 | 5 | | 5 | 5 | 0 | 0 | 3 | 0 | 0 | 1 |
| | 1 | 5 | 5 | | 5 | 5 | 0 | 0 | 1 | 0 | 0 | 1 |
| | 0.5 | 4 | 5 | | 5 | 5 | 0 | 0 | 0 | 0 | 0 | |
| 8 | 2 | 5 | 5 | 5 | 5 | 5 | 3.5 | 3 | 5 | 0 | 0 | 4.5 |
| | 1 | 5 | 5 | 5 | 5 | 5 | 1.5 | 2 | 3.5 | 0 | 0 | 3.5 |
| | 0.5 | 5 | 5 | 5 | 5 | 5 | 0 | 1 | 1 | 0 | 0 | 2.5 |

(to be continued)

Table 5C (continued)

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar-beet |
| 12 | 2 | 5 | 5 | 5 | 5 | 5 | 0.5 | 1 | 2 | 0 | 0 | 2 |
| | 1 | 2.5 | 5 | 4 | 5 | 5 | 0 | 0 | 0 | 0 | 0 | 1.5 |
| | 0.5 | 1 | 4 | 2.5 | 5 | 5 | 0 | 0 | 0 | 0 | 0 | 0.5 |
| 13 | 2 | 5 | 5 | 5 | 5 | 5 | 0 | 1 | 2 | 0 | 0 | 1 |
| | 1 | 5 | 5 | 5 | 5 | 5 | 0 | 1 | 1 | 0 | 0 | 1 |
| | 0.5 | 5 | 5 | 5 | 4 | 5 | 0 | 0 | 1 | 0 | 0 | 0 |
| 14 | 2 | 5 | 5 | | 5 | 5 | 4 | 3 | 4 | 0 | 0 | 2 |
| | 1 | 5 | 5 | | 5 | 5 | 1 | 1 | 3 | 0 | 0 | 2 |
| | 0.5 | 5 | 5 | | 5 | 5 | 0 | 0 | 2 | 0 | 0 | 1 |
| 15 | 2 | 5 | 5 | 5 | 5 | 5 | 1 | 1 | 2 | 1 | 1 | 2 |
| | 1 | 5 | 5 | 5 | 5 | 5 | 1 | 1 | 2 | 0 | 0 | 1 |
| | 0.5 | 5 | 5 | 5 | 5 | 5 | 0 | 0 | 0 | 0 | 0 | 0 |
| 21 | 2 | 5 | 5 | | 5 | 5 | 1 | 1 | 2 | 0 | | 1 |
| | 1 | 5 | 5 | | 5 | 5 | 0 | 1 | 2 | 0 | | 1 |
| | 0.5 | 5 | 5 | | 5 | 5 | 0 | 0 | 0 | 0 | | 1 |

(to be continued)

Table 5C (continued)

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar-beet |
| 22 | 2 | 5 | 5 | | 5 | 5 | 0 | 0 | 2 | 0 | 0 | 1 |
| | 1 | 5 | 5 | | 5 | 5 | 0 | 0 | 0 | 0 | 0 | 0 |
| | 0.5 | 5 | 5 | | 5 | 5 | 0 | 0 | 0 | 0 | 0 | |
| 23 | 2 | 5 | 5 | | 5 | 5 | 1 | 0 | 0 | 0 | 0 | 0 |
| | 1 | 5 | 5 | | 5 | 5 | 0 | 0 | 0 | 0 | 0 | 0 |
| | 0.5 | 5 | 5 | | 3 | 5 | 0 | 0 | 0 | 0 | 0 | 0 |
| 24 | 2 | 5 | 5 | | 5 | 5 | 0 | 1 | 2 | 0 | 1 | 1 |
| | 1 | 5 | 5 | | 5 | 5 | 0 | 0 | 1 | 0 | | 1 |
| | 0.5 | 5 | 5 | | 5 | 5 | 0 | 0 | 0 | 0 | 0 | 0 |
| 25 | 2 | 5 | 5 | 5 | 2 | 5 | 0 | 0 | 1 | 0 | 0 | 0 |
| | 1 | 5 | 5 | 4.5 | 1 | 5 | 0 | 0 | 0.5 | 0 | 0 | 0 |
| | 0.5 | 4 | 5 | 4 | 0 | 5 | 0 | 0 | 0 | 0 | 0 | 0 |
| 33 | 2 | 5 | 5 | 5 | 5 | 5 | 2 | 3 | 4 | 0 | 1 | 3 |
| | 1 | 5 | 5 | 5 | 5 | 5 | 0 | 1 | 4 | 0 | 0 | 2 |
| | 0.5 | 5 | 5 | 5 | 5 | 5 | 0 | 1 | 3 | 0 | 0 | 0 |

(to be continued)

Table 5C (continued)

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar-beet |
| 34 | 2 | 5 | 5 | 5 | 5 | 5 | 3 | 4 | 5 | 0 | 0 | 3 |
| | 1 | 5 | 5 | 5 | 5 | 5 | 1 | 2 | 4 | 0 | 0 | 2 |
| | 0.5 | 5 | 5 | 5 | 5 | 5 | 0 | 0 | 3 | 0 | 0 | 1 |
| 39 | 2 | 5 | 5 | 5 | 3 | 5 | 4.5 | 3 | 5 | 0 | 0 | 3 |
| | 1 | 5 | 5 | 5 | 1 | 5 | 3.5 | 3 | 5 | 0 | 0 | 2.5 |
| | 0.5 | 5 | 5 | 5 | 0 | 5 | 3 | 1 | 3 | 0 | 0 | 1 |
| 40 | 2 | 5 | 5 | 5 | 3 | 5 | 4.5 | 3 | 5 | 0 | 0 | 1.5 |
| | 1 | 5 | 5 | 5 | 1 | 5 | 2.5 | 1.5 | 4.5 | 0 | 0 | 1 |
| | 0.5 | 5 | 5 | 5 | 0 | 5 | 2 | 1 | 4.5 | 0 | 0 | 0.5 |
| 42 | 2 | 5 | 5 | | 5 | 5 | 1 | 2 | 3 | 0 | | 1 |
| | 1 | 5 | 5 | | 5 | 5 | 1 | 1 | 2 | 0 | 0 | 1 |
| | 0.5 | 5 | 5 | | 5 | 5 | 0 | 0 | 1 | 0 | 0 | 0 |
| 43 | 2 | 5 | 5 | 5 | 5 | 5 | 0.5 | 1 | 2 | 0 | 0 | 1.5 |
| | 1 | 5 | 5 | 4.5 | 3 | 5 | 0 | 0.5 | 1.5 | 0 | 0 | 1 |
| | 0.5 | 3.5 | 5 | 4.5 | 4 | 5 | 0 | 0 | 1 | 0 | 0 | 0.5 |

(to be continued)

Table 5C (continued)

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar-beet |
| 45 | 2 | 5 | 5 | 5 | 2 | 5 | 1.5 | 1 | 3.5 | 0 | 0 | 1.5 |
| | 1 | 5 | 4.5 | 4.5 | 2 | 5 | 0.5 | 0.5 | 0.5 | 0 | 0 | 1 |
| | 0.5 | 3.5 | 4.5 | 4.5 | 1 | 5 | 0 | 0 | 0 | 0 | 0 | 0.5 |
| 53 | 2 | 5 | 5 | 5 | 5 | 5 | 2 | 2.5 | 5 | 1 | 1 | 2 |
| | 1 | 5 | 5 | 5 | 5 | 5 | 1.5 | 1.5 | 5 | 0 | 0.5 | 1.5 |
| | 0.5 | 5 | 5 | 5 | 0 | 5 | 1 | 1.5 | 5 | 0 | 0 | 1 |
| 58 | 2 | 5 | 5 | 5 | 5 | 5 | 0.5 | 0.5 | 1.5 | 0 | 0 | 1.5 |
| | 1 | 4.5 | 4.5 | 5 | 5 | 5 | 0 | 0 | 0.5 | 0 | 0 | 1 |
| | 0.5 | 2.5 | 4.5 | 4.5 | 5 | 5 | 0 | 0 | 0 | 0 | 0 | 0.5 |

Test Example 5C (herbicidal test by foliar treatment in an upland farm

Upland farm soil was filled in plastic pots (120 $cm^2$), and seeds of barnyardgrass, crabgrass, redroot pigweed and rice flatsedge were sown, and grown in a greenhouse until the barnyardgrass reached a three-leaf stage. Each of the test compounds was formulated into a wettable powder in accordance with Formulation Example 3B. The wettale powder in an amount equivalent to 4 kg per hectare as the active

ingredient was diluted with water, and sprayed over the foliage of the plants from above by a small-sized sprayer at a rate of 1000 liters per hectare. After the spraying, the plants were grown in a greenhouse for 20 days. The herbicidal efficacy was examined in accordance with the standards given in Table 1C, and the results are shown in Table 6C.

Table 6C

| Test compound No. | Herbicidal efficacy | | | |
|---|---|---|---|---|
| | barnyard-grass | crab-grass | redroot pigweed | rice flatsedge |
| 1 | 5 | 5 | 1 | 4 |
| 2 | 5 | 5 | 4 | 4 |
| 3 | 5 | 5 | 5 | 5 |
| 4 | 2 | 5 | 4 | 5 |
| 5 | 5 | 5 | 5 | 5 |
| 6 | 5 | 5 | 3 | 5 |
| 7 | 4 | 4 | 2 | 4 |
| 8 | 5 | 5 | 2 | 4 |
| 9 | 4 | 5 | 3 | 5 |
| 11 | 4 | 5 | 2 | 5 |
| 12 | 5 | 5 | 1 | 3 |
| 13 | 5 | 5 | 5 | 5 |
| 14 | 5 | 5 | 5 | 5 |
| 15 | 5 | 5 | 4 | 5 |
| 16 | 5 | 5 | 5 | 5 |

- to be continued -

Table 6C (continued)

| Test compound No. | Herbicidal efficacy | | | |
|---|---|---|---|---|
| | barnyard-grass | crab-grass | redroot pigweed | rice flatsedge |
| 17 | 5 | 5 | 0 | 4 |
| 18 | 5 | 5 | 2 | 4 |
| 19 | 5 | 5 | 4 | 5 |
| 20 | 5 | 5 | 3 | 5 |
| 21 | 5 | 5 | 5 | 5 |
| 22 | 5 | 5 | 5 | 5 |
| 23 | 5 | 5 | 5 | 5 |
| 24 | 5 | 5 | 4 | 5 |
| 25 | 5 | 5 | 3 | 5 |
| 26 | 4 | 4 | 3 | 5 |
| 27 | 4 | 4 | 2 | 4 |
| 33 | 5 | 5 | 5 | 5 |
| 34 | 5 | 5 | 5 | 5 |
| 39 | 5 | 5 | 3 | 5 |
| 40 | 5 | 5 | 3 | 5 |

- to be continued -

Table 6C (continued)

| Test compound No. | Herbicidal efficacy | | | |
|---|---|---|---|---|
| | barnyard-grass | crab-grass | redroot pigweed | rice flatsedge |
| 42 | 5 | 5 | 5 | 5 |
| 43 | 5 | 5 | 3 | 5 |
| 45 | 5 | 5 | 2 | 5 |
| 46 | 5 | 4 | 3 | 5 |
| 47 | 5 | 4 | 3 | 5 |
| 49 | 4 | 4 | 0 | 5 |
| 50 | 4 | 4 | 0 | 5 |
| 51 | 4 | 4 | 2 | 5 |
| 52 | 4 | 4 | 0 | 5 |
| 53 | 5 | 5 | 4 | 5 |
| 54 | 5 | 5 | 2 | 5 |
| 55 | 5 | 5 | 3 | 5 |
| 56 | 5 | 5 | 3 | 5 |
| 57 | 5 | 5 | 4 | 5 |
| 58 | 5 | 5 | 2 | 5 |

- to be continued -

Table 6C (continued)

| Test compound No. | Herbicidal efficacy | | | |
|---|---|---|---|---|
| | barnyard-grass | crab-grass | redroot pigweed | rice flatsedge |
| 59 | 5 | 5 | 4 | 5 |
| 60 | 5 | 5 | 3 | 5 |
| 61 | 5 | 5 | 2 | 5 |
| 62 | 5 | 5 | 4 | 5 |
| 63 | 5 | 4 | 3 | 5 |
| 65 | 5 | 5 | 2 | 5 |
| 66 | 5 | 5 | 2 | 5 |
| 67 | 5 | 5 | 2 | 5 |
| 68 | 5 | 5 | 2 | 5 |
| 73 | 5 | 3 | 3 | 5 |
| 76 | 5 | 5 | 4 | 5 |
| 77 | 5 | 5 | 2 | 5 |
| 81 | 5 | 5 | 4 | 5 |

Test Example 6C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied: 4 kg per hectare as the active ingredient). The plants were then grown in a greenhouse, and 30 days after the treatment, the herbicidal activity of the compound of the invention was examined according to the standards given in Table 1C. The results are shown in Table 7C.

Table 7C

| Test compound No. | Herbicidal efficacy | | | |
|---|---|---|---|---|
| | barnyardgrass | umbrella plant | monochoria | bulrush |
| 82 | 5 | 5 | 5 | 5 |
| 83 | 5 | 3 | 5 | 3 |
| 84 | 5 | 5 | 5 | 5 |
| 85 | 2 | 2 | 0 | 1 |
| 86 | 5 | 5 | 5 | 5 |
| 87 | 5 | 5 | 5 | 5 |
| 88 | 5 | 5 | 5 | 5 |
| 89 | 5 | 5 | 5 | 5 |
| 90 | 5 | 5 | 5 | 5 |
| 91 | 5 | 5 | 5 | 5 |
| 92 | 5 | 5 | 5 | 5 |
| 93 | 5 | 5 | 5 | 5 |
| 94 | 5 | 5 | 5 | 5 |
| 95 | 2 | 5 | 5 | 2 |

Test Example 7C (herbicidal test by soil treatment in an upland farm)

Upland farm soil was filled in plastic pots (120 $cm^2$), and seeds of barnyardgrass, crabgrass, redroot pigweed and rice flatsedge were sown and covered with the soil. Each of the test compounds was formulated into a wettable powder in accordance with Formulation Example 3B. A predetermined amount of the wettable powder was diluted with water, and by a small-sized sprayer, uniformly sprayed onto the soil surface at a rate of 1000 liters/hectare (the amount applied: 4 kg per hectare as the active ingredient). The plants were grown for 20 days in a greenhouse after the treatment, and the herbicidal efficacy and phytotoxicity were examined in accordance with the standards given in Table 1C. The results are shown in Table 8C.

Table 8C

| Test compound No. | Herbicidal efficacy | | | |
|---|---|---|---|---|
| | barnyardgrass | crabgrass | redroot pigweed | rice flatsedge |
| 82 | 5 | 5 | 3 | 5 |
| 83 | 1 | 4 | 2 | 5 |
| 84 | 5 | 5 | 2 | 5 |
| 85 | 1 | 2 | 2 | 5 |
| 86 | 5 | 5 | 5 | 5 |
| 87 | 5 | 5 | 5 | 5 |
| 88 | 5 | 5 | 4 | 5 |
| 89 | 5 | 5 | 5 | 5 |
| 90 | 5 | 5 | 5 | 5 |
| 91 | 0 | 3 | 2 | 5 |
| 92 | 5 | 5 | 5 | 5 |
| 93 | 5 | 5 | 5 | 5 |
| 94 | 5 | 5 | 3 | 5 |
| 95 | 1 | 1 | 2 | 3 |

Test Example 8C (herbicidal efficacy and phytotoxicity by soil treatment in an upland farm

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar beet were sown and covered with the soil. Each of the test compounds was formulated into a wettable powder in accordance with Formulation Example 3B. A predetermined amount of the wettable powder was diluted with water and uniformly sprayed onto the soil surface by a small-sized sprayer at a rate of 1000 liters per hectare. The plants were grown in a greenhouse for 30 days after the treatment. The herbicidal efficacy and phytotoxicity were examined in accordance wiht the standards given in Table 1C. The results are shown in Table 9C.

Table 9C

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar beet |
| 82 | 1 | 5 | 5 | 5 | - | 5 | 1 | 0 | 2 | 0.5 | 0 | 1 |
| | 0.5 | 5 | 5 | 5 | - | 5 | 0 | 0 | 2 | 0 | 0 | 0.5 |
| | 0.25 | 5 | 5 | 5 | - | 5 | 0 | 0 | 1.5 | 0 | 0 | 0.5 |
| 84 | 4 | 5 | 5 | 5 | - | 5 | 0.5 | 0.5 | 5 | 0.5 | 0.5 | 1 |
| | 2 | 5 | 5 | 5 | - | 5 | 0.5 | 0 | 3.5 | 0 | 0 | 0.5 |
| | 1 | 5 | 5 | 5 | - | 5 | 0 | 0 | 2 | 0 | 0 | 0.5 |
| 86 | 1 | 5 | 5 | 5 | 5 | 5 | 1 | 0.5 | 2 | 0 | 1 | 4 |
| | 0.5 | 5 | 5 | 5 | 5 | 5 | 0 | 0 | 1.5 | 0 | 0 | 4 |
| | 0.25 | 5 | 5 | 5 | 4.5 | 5 | 0 | 0 | 1.5 | 0 | 0 | 4 |

- to be continued -

Table 9C (continued)

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar beet |
| 89 | 1 | 5 | 5 | 5 | 5 | 5 | 4.5 | 2.5 | 5 | 0 | 0 | 0.5 |
| | 0.5 | 5 | 5 | 5 | 5 | 5 | 3 | 2 | 5 | 0 | 0 | 0 |
| | 0.25 | | 5 | 5 | 5 | 5 | 2 | 2 | 4.5 | 0 | 0 | 0 |
| 92 | 1 | 5 | 5 | 5 | 5 | 5 | 2.5 | 1 | 1.5 | 0 | 0 | 1.5 |
| | 0.5 | 5 | 5 | 5 | 4 | 5 | 2 | 0.5 | 1 | 0 | 0 | 1.5 |
| | 0.25 | 5 | 5 | 5 | 3 | 5 | 1 | 0 | 0 | 0 | 0 | 1.5 |
| 93 | 1 | 5 | 5 | 5 | 1 | 5 | 0 | 0 | 1.5 | 0.5 | 0 | 0 |
| | 0.5 | 5 | 5 | 5 | 1 | 5 | 0 | 0 | 1 | 0 | 0 | 0 |
| | 0.25 | 5 | 5 | 5 | 0 | 5 | 0 | 0 | 0 | 0 | 0 | 0 |
| 94 | 2 | 5 | 5 | 5 | 2 | 5 | 0 | 0.5 | 0.5 | 1 | 1 | 1.5 |
| | 1 | 5 | 5 | 5 | 1.5 | 5 | 0 | 0 | 0 | 0 | 0 | 0 |
| | 0.5 | 4.5 | 4.5 | 3.5 | 0.5 | 5 | 0 | 0 | 0 | 0 | 0 | 0 |

Test Example 9C (herbicidal test by soil treatment in a paddy)

Porcelain pots, 10 cm in diameter, were filled with paddy soil, and after puddling, seeds of barnyardgrass, umbrella plant, monochoria and bulrush were sown. The pots were then watered to a depth of 3 cm. On the next day, a wettable powder prepared in accordance with Formulation Example 3B was diluted with water and dropped onto the water surface (amount applied: 4 kg per hectare as the active ingredient). The

plants were then grown in a greenhouse, and 30 days after the treatment, the herbicidal activity of the compound of the invention was examined according to the standards given in Table 1C. The results are shown in Table 10C.

Table 10C

| Test compound No. | Herbicidal efficacy | | | |
|---|---|---|---|---|
| | barnyardgrass | umbrella plant | monochoria | bulrush |
| 113 | 5 | 5 | 5 | 5 |
| 116 | 5 | 5 | 5 | 5 |
| 117 | 5 | 5 | 5 | 4 |
| 118 | 5 | 5 | 5 | 4 |
| 119 | 5 | 5 | 5 | 5 |
| 165 | 5 | 4 | 4 | 4 |
| 166 | 4 | 3 | 2 | 4 |

Test Example 10C (herbicidal test by soil treatment in an upland farm)

Upland farm soil was filled in plastic vats (600 $cm^2$). Seeds of barnyardgrass, crabgrass, green foxtail, redroot pigweed, rice flatsedge, rice, wheat, corn, soybeans, cotton and sugar beet were sown and covered with the soil. Each of the test compounds was formulated into a wettable powder in accordance with Formulation Example 3B. A predetermined amount of the wettable powder was diluted with water and uniformly sprayed onto the soil surface by a small-sized sprayer at a rate of 1000 liters per hectare. The plants were grown in a greenhouse for 30 days after the treatment. The herbicidal efficacy and phytotoxicity were examined in accordance with the standards given in Table 1C. The results are shown in Table 11C.

Table 11C

| Test compound No. | Amount applied (kg/ha) | Herbicidal efficacy | | | | | Phytotoxicity | | | | | |
|---|---|---|---|---|---|---|---|---|---|---|---|---|
| | | barnyard-grass | crab-grass | green foxtail | redroot pigweed | rice flatsedge | rice | wheat | corn | soy beans | cotton | sugar beet |
| 119 | 1 | 5 | 5 | 5 | 5 | 5 | 0.5 | 0 | 4.5 | 0 | 3 | 3 |
| | 0.5 | 5 | 5 | 5 | 5 | 5 | 0 | 0 | 2 | 0 | 1 | 3 |
| | 0.25 | 5 | 5 | 5 | 5 | 5 | 0 | 0 | 0 | 0 | 0 | 2 |

**Claims**

**Claims for the following Contracting States : BE, CH, DE, FR, GB, IT, LI, NL**

1. An aryloxyurea represented by the following formula (I)

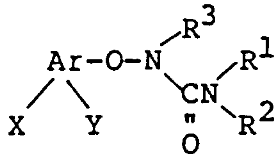

..... (I)

wherein

Ar represents a group selected from the class consisting of phenyl, pyridyl, pyridazinyl, pyrimidinyl and pyrazinyl groups,

X and Y are identical or different and each represents a hydrogen or halogen atom, or a cyano, trifluoromethyl, nitro, lower alkoxy, lower alkylthio or lower alkoxycarbonyl group,

$R^1$ represents a hydrogen atom, or a lower alkyl, lower alkenyl, lower alkynyl or $C_3$-$C_7$ cycloalkyl group,

$R^2$ represents a hydrogen atom, or a lower alkyl,

$R^1$ and $R^2$, taken together, may form a 4- to 8-membered heterocyclic group together with the nitrogen atom to which they are bonded, wherein the heterocyclic group may contain an oxygen atom as a ring-member atom or may be substituted by a lower alkyl group or a lower alkylene group,

$R^3$ represents a group of the formula $-COR^4$, a group of the formula $-CH_2OR^5$, or a lower alkyl group,

$R^4$ represents a hydrogen atom, or a lower alkyl, lower haloalkyl, lower alkoxymethyl, lower alkoxy, lower alkoxy-substituted lower alkoxy or halogen-substituted lower alkoxy group, and

$R^5$ represents a hydrogen atom or a lower alkyl group; and

an acid addition salt thereof.

**2.** The compounds set forth in claim 1 wherein in formula (I), X and Y, independently from each other, represent a hydrogen or chlorine atom or a trifluoromethyl group.

**3.** The compounds set forth in claim 1 wherein in formula (I), Ar is a phenyl or pyridyl group.

**4.** The compounds set forth in claim 1 wherein in formula (I), $R^1$ and $R^2$, taken together, form a 6- or 7-membered heterocyclic group together with the nitrogen atom to which they are bonded.

**5.** The compounds set forth in claim 1 wherein in formula (I),

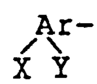

is 3,5-dichlorophenyl, or 3-chloro-5-trifluoromethyl-2-pyridyl,

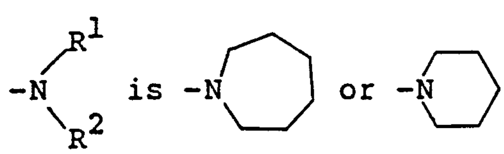

is

or

and $R^3$ is ethoxycarbonyl, n-propoxycarbonyl, or isopropoxycarbonyl.

**6.** A process for producing an aryloxyurea represented by the following formula (I)-1

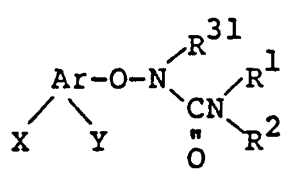

(I)-1

wherein Ar, X, Y, $R^1$ and $R^2$ are as defined with regard to formula (I), and $R^{31}$ represents a group of the formula $-COR^{41}$, a group of the formula $-CH_2OR^{51}$ or a lower alkyl group,

$R^{41}$ represents a lower alkyl, lower haloalkyl, lower alkoxymethyl, lower alkoxy, lower alkoxy-substituted lower alkoxy or halogen-substituted lower alkoxy group, and $R^{51}$ represents a lower alkyl group,

which comprises reacting a compound represented by the following formula (II)

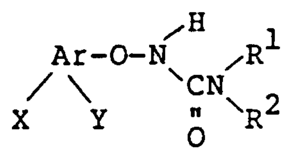

(II)

wherein Ar, X, Y, $R^1$ and $R^2$ are as defined with regard to formula (I),
with a compound represented by the following formula (III)

$R^{31}$-Cl (III)

wherein $R^{31}$ is as defined with regard to formula (I)-1,
in the presence of a base.

**7.** A process for producing an aryloxyurea represented by the following formula (I)-2

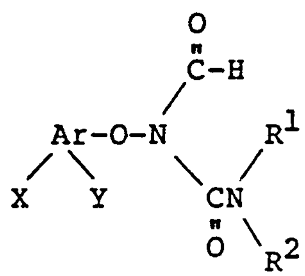

(I)-2

wherein Ar, X, Y, $R^1$ and $R^2$ are as defined with regard to formula (I), which comprises reacting a compound represented by the following formula (II)

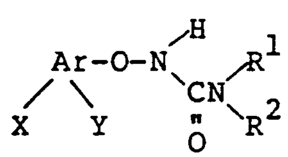

(II)

wherein Ar, X, Y, $R^1$ and $R^2$ are as defined with regard to formula (I),
with a compound represented by the following formula (IV)

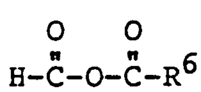

(IV)

wherein $R^6$ represents a lower alkyl group.

**8.** A process for producing an aryloxyurea represented by the following formula (I)-3

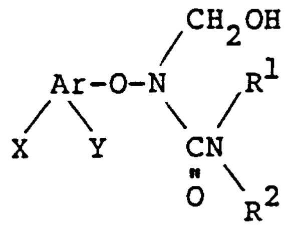

(I)-3

wherein Ar, X, Y, $R^1$ and $R^2$ are as defined with regard to formula (I), which comprises reacting a compound represented by the following formula (II)

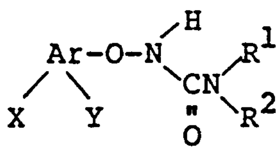

(II)

wherein Ar, X, Y, $R^1$ and $R^2$ are as defined with regard to formula (I), with formaldehyde.

**9.** A process for producing an aryloxyurea represented by the following formula (I)-4

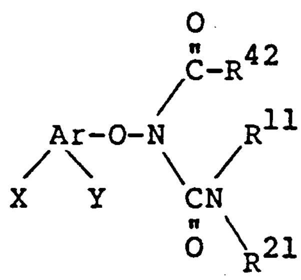

(I)-4

wherein Ar, X and Y are as defined with regard to formula (I), $R^{11}$ represents a lower alkyl, lower alkenyl, lower alkynyl or $C_3$-$C_7$cycloalkyl group, $R^{21}$ represents a lower alkyl group, and $R^{11}$ and $R^{21}$, taken together, may form a 4- to 8-membered heterocyclic group together with the nitrogen atom to which they are bonded, said heterocylic group optionally containing an oxygen atom as a ring-member atom or being substituted by a lower alkyl or alkylene group, and $R^{42}$ represents a lower alkoxy, lower alkoxy-substituted lower alkoxy, or halogen-substituted lower alkoxy group, which comprises reacting a compound represented by the following formula (V)

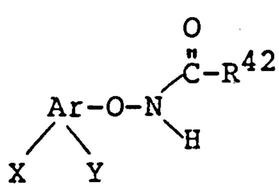

(V)

wherein Ar, X and Y are as defined with regard to formula (I), and $R^{42}$ is as defined with regard to formula (I)-4, with a carbamoyl chloride represented by the following formula (VI)

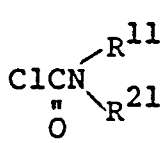

(VI)

wherein $R^{11}$ and $R^{21}$ are as defined with regard to formula (I)-4.

**10.** A process for producing an aryloxyurea represented by the following formula (I)-5

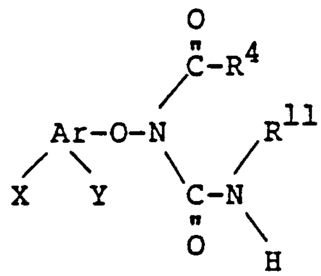

(I)-5

wherein Ar, X, Y, $R^1$ and $R^4$ are as defined hereinabove with regard to formula (I), which comprises reacting a compound represented by the following formula (VII)

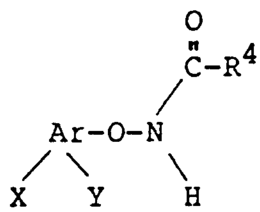

(VII)

wherein Ar, X, Y and $R^4$ are as defined above with respect to formula (I), with an isocyanate ester represented by the following formula (VIII)

$O=C=N-R^{12}$ (VIII)

wherein $R^{12}$ represents a lower alkyl, lower alkenyl, lower alkynyl or $C_3$-$C_7$ cycloalkyl group.

**11.** A process for producing an aryloxyurea represented by the following formula (I)-6

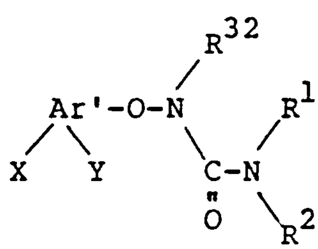

(I)-6

which comprises reacting a compound represented by the following formula (IX)

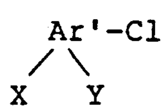

(IX)

wherein Ar' is a group selected from pyrimidinyl and pyridazinyl groups, and $R^{32}$ represents a lower alkyl group,
with an N-hydroxyurea represented by the following formula (X)

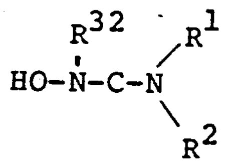

(X)

wherein $R^1$ and $R^2$ are as defined with regard to formula (I), and $R^{32}$ is as defined with regard to formula (X), in the presence of a base.

**12.** A herbicide comprising an aryloxyurea represented by formula (I) of claim 1 or its acid addition salt as an active ingredient.

**Claims for the following Contracting State : AT**

**1.** A process for producing an aryloxyurea represented by the following formula (I)

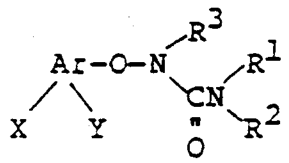

..... (I)

wherein

Ar represents a group selected from the class consisting of phenyl, pyridyl, pyridazinyl, pyrimidinyl and pyrazinyl groups,

X and Y are identical or different and each represents a hydrogen or halogen atom, or a cyano, trifluoromethyl, nitro, lower alkoxy, lower alkylthio or lower alkoxycarbonyl group,

$R^1$ represents a hydrogen atom, or a lower alkyl, lower alkenyl, lower alkynyl or $C_3$-$C_7$ cycloalkyl group,

$R^2$ represents a hydrogen atom, or a lower alkyl,

$R^1$ and $R^2$, taken together, may form a 4- to 8-membered heterocyclic group together with the nitrogen atom to which they are bonded, wherein the heterocyclic group may contain an oxygen atom as a ring-member atom or may be substituted by a lower alkyl group or a lower alkylene group,

$R^3$ represents a group of the formula $-COR^4$, a group of the formula $-CH_2OR^5$, or a lower alkyl group,

$R^4$ represents a hydrogen atom, or a lower alkyl, lower haloalkyl, lower alkoxymethyl, lower alkoxy, lower alkoxy-substituted lower alkoxy or halogen-substituted lower alkoxy group, and

$R^5$ represents a hydrogen atom or a lower alkyl group; and

an acid addition salt thereof;

wherein for producing an aryloxyurea represented by the following formula (I)-1

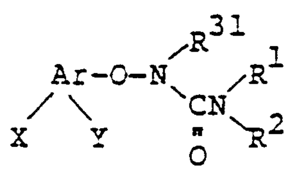

(I)-1

wherein Ar, X, Y, $R^1$ and $R^2$ are as defined with regard to formula (I), and $R^{31}$ represents a group of the formula $-COR^{41}$, a group of the formula $-CH_2OR^{51}$ or a lower alkyl group,

$R^{41}$ represents a lower alkyl, lower haloalkyl, lower alkoxymethyl, lower alkoxy, lower alkoxysubstituted lower alkoxy or halogen-substituted lower alkoxy group, and $R^{51}$ represents a lower alkyl group, a compound represented by the following formula (II)

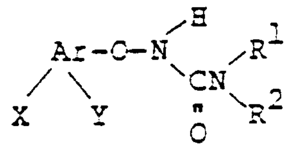

(II)

wherein Ar, X, Y, $R^1$ and $R^2$ are as defined with regard to formula (I), is reacted with a compound represented by the following formula (III)

$R^{31}$-Cl (III)

wherein $R^{31}$ is as defined with regard to formula (I)-1, in the presence of a base;
or wherein for producing an aryloxyurea represented by the following formula (I)-2

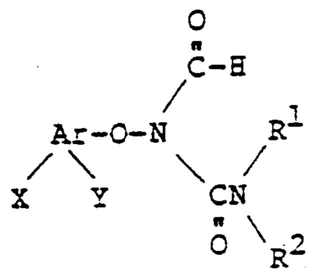

(I)-2

wherein Ar, X, Y, $R^1$ and $R^2$ are as defined with regard to formula (I), a compound represented by the following formula (II)

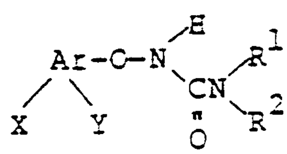

(II)

wherein Ar, X, Y, $R^1$ and $R^2$ are as defined with regard to formula (I), is reacted with a compound represented by the following formula (IV)

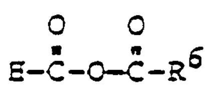

(IV)

wherein $R^6$ represents a lower alkyl group;
or wherein for producing an aryloxyurea represented by the following formula (I)-3

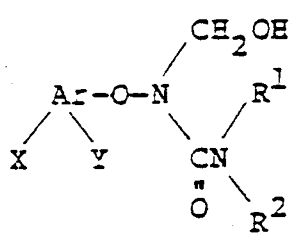

(I)-3

wherein Ar, X, Y, $R^1$ and $R^2$ are as defined with regard to formula (I), a compound represented by the following formula (II)

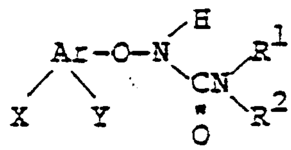

(II)

wherein Ar, X, Y, $R^1$ and $R^2$ are as defined with regard to formula (I), is reacted with formaldehyde; or wherein for producing an aryloxyurea represented by the following formula (I)-4

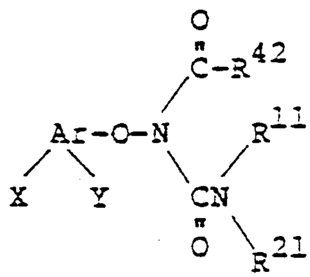

(I)-4

wherein Ar, X and Y are as defined with regard to formula (I), $R^{11}$ represents a lower alkyl, lower alkenyl, lower alkynyl or $C_3$-$C_7$cycloalkyl group, $R^{21}$ represents a lower alkyl group, and $R^{11}$ and $R^{21}$, taken together, may form a 4- to 8-membered heterocyclic group together with the nitrogen atom to which they are bonded, said heterocylic group optionally containing an oxygen atom as a ring-member atom or being substituted by a lower alkyl or alkylene group, and $R^{42}$ represents a lower alkoxy, lower alkoxy-substituted lower alkoxy, or halogen-substituted lower alkoxy group, a compound represented by the following formula (V)

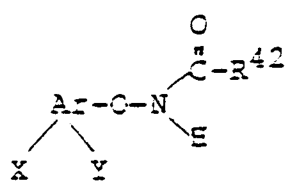

(V)

wherein Ar, X and Y are as defined with regard to formula (I), and $R^{42}$ is as defined with regard to formula (I)-4, is reacted with a carbamoyl chloride represented by the following formula (VI)

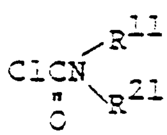

(VI)

wherein $R^{11}$ and $R^{21}$ are as defined with regard to formula (I)-4 ; or wherein for producing an aryloxyurea represented by the following formula (I)-5

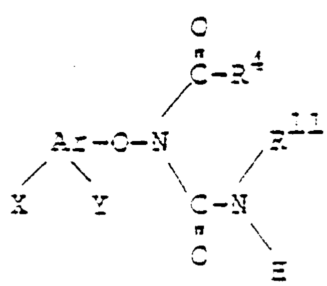

(I)-5

wherein Ar, X, Y, $R^1$ and $R^4$ are as defined hereinabove with regard to formula (I), a compound represented by the following formula (VII) following formula (VII)

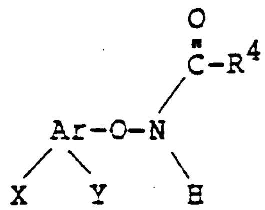

(VII)

wherein Ar, X, Y and $R^4$ are as defined above with respect to formula (I), is reacted with an isocyanate ester by the following formula (VIII)

$O=C=N\text{-}R^{12}$ (VIII)

wherein $R^{12}$ represents a lower alkyl, lower alkenyl, lower alkynyl or $C_3$-$C_7$ cycloalkyl group ; or wherein for producing an aryloxyurea represented by the following formula (I)-6

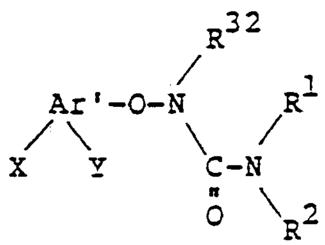

(I)-6

a compound represented by the following formula (IX)

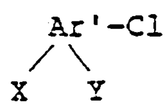

(IX)

wherein Ar' is a group selected from pyrimidinyl and pyridazinyl groups, and $R^{32}$ represents a lower alkyl group,
is reacted with an N-hydroxyurea represented by the following formula (X)

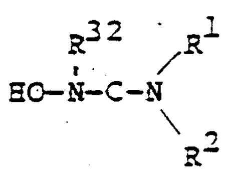

(X)

wherein $R^1$ and $R^2$ are as defined with regard to formula (I), and $R^{32}$ is as defined with regard to formula (X),
in the presence of a base.

**2.** The process set forth in claim 1 wherein in formula (I), X and Y, independently from each other, represent a hydrogen or chlorine atom or a trifluoromethyl group.

**3.** The process set forth in claim 1 wherein in formula (I), Ar is a phenyl or pyridyl group.

**4.** The process set forth in claim 1 wherein in formula (I), $R^1$ and $R^2$, taken together, form a 6- or 7-membered heterocyclic group together with the nitrogen atom to which they are bonded.

5. The process set forth in claim 1 wherein in formula (I),

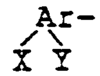

is 3,5-dichlorophenyl, or 3-chloro-5-trifluoromethyl-2-pyridyl,

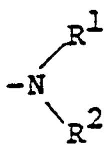 is 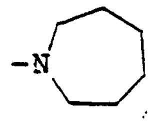 or 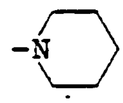

and $R^3$ is ethoxycarbonyl, n-propoxycarbonyl, or isopropoxycarbonyl.

6. A herbicide comprising an aryloxyurea represented by formula (I) of Claim 1 or its acid addition salt as an active ingredient.

## Patentansprüche

**Patentansprüche für folgende Vertragsstaaten : BE, CH, DE, FR, GB, IT, LI, NL**

1. Aryloxyharnstoff folgender Formel (I)

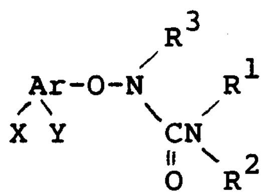

(I)

in der Ar eine Gruppe repräsentiert, welche aus der Klasse, die aus Phenyl-, Pyridyl-, Pyridazinyl-, Pyrimidinyl- und Pyrazinylgruppen besteht, ausgewählt wird,

X und Y identisch oder unterschiedlich sind und je für ein Wasserstoff- oder Halogenatom oder für eine Cyano-, Trifluormethyl-, Nitro-, niedere Alkoxy-, niedere Alkylthio- oder niedere Alkoxycarbonylgruppe stehen,

$R^1$ ein Wasserstoffatom oder eine niedere Alkyl-, niedere Alkenyl-, niedere Alkinyl- oder eine $C_3$-$C_7$-Cycloalkylgruppe bedeutet,

$R^2$ für ein Wasserstoffatom oder eine niedere Alkylgruppe steht,

$R^1$ und $R^2$, zusammengefaßt, zusammen mit dem Stickstoffatom, an das sie gebunden sind, eine 4- bis 8-gliedrige heterocyclische Gruppe bilden können, wobei die heterocyclische Gruppe ein Sauerstoffatom als ein Ringbestandteil enthalten kann oder durch eine niedere Alkylgruppe oder eine niedere Alkylengruppe substituiert sein kann,

$R^3$ für eine Gruppe der Formel $-COR^4$, eine Gruppe der Formel $-CH_2OR^5$, oder für eine niedere Alkylgruppe steht,

$R^4$ ein Wasserstoffatom oder eine niedere Alkyl-, niedere Halogenalkyl-, niedere Alkoxymethyl-, niedere Alkoxy-, nieder-alkoxysubstituierte niedere Alkoxy- oder eine halogensubstituierte niedere Alkoxygruppe bedeutet, und

$R^5$ für ein Wasserstoffatom oder eine niedere Alkylgruppe steht; und

ein Säureadditionssalz desselben.

2. Verbindungen nach Anspruch 1, wobei in Formel (I) X und Y unabhängig voneinander für ein Wasserstoff- oder ein Chloratom oder für eine Trifluormethylgruppe stehen.

3. Verbindungen nach Anspruch 1, wobei in Formel (I) Ar eine Phenyl- oder Pyridylgruppe ist.

4. Verbindungen nach Anspruch 1, wobei in Formel (I) $R^1$ und $R^2$, zusammengefaßt, zusammen mit dem Stickstoffatom, an das sie gebunden sind, eine 6- oder 7-gliedrige heterocyclische Gruppe bilden.

**5.** Verbindungen nach Anspruch 1, wobei in Formel (I)

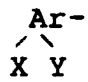

für 3,5-Dichlorphenyl oder 3-Chlor-5-trifluormethyl-2-pyridyl steht,

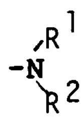 für 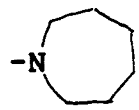

oder

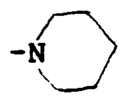

steht,
und $R^3$ für Ethoxycarbonyl, n-Propoxycarbonyl oder für Isopropoxycarbonyl steht.

**6.** Verfahren zur Herstellung eines Aryloxyharnstoffs der folgenden Formel (I)-1

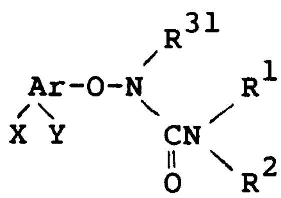

(I)-1

in der Ar, X, Y, $R^1$ und $R^2$ wie bei Formel (I) definiert sind und $R^{31}$ für eine Gruppe der Formel $-COR^{41}$, eine Gruppe der Formel $-CH_2OR^{51}$oder für eine niedere Alkylgruppe steht, $R^{41}$ eine niedere Alkyl-, niedere Halogenalkyl-, niedere Alkoxymethyl-, niedere Alkoxy-, nieder-alkoxysubstituierte niedere Alkoxy- oder eine halogensubstituierte niedere Alkoxygruppe bedeutet, und $R^{51}$ für eine niedere Alkylgruppe steht,
folgendes umfassend: das Umsetzen einer Verbindung folgender Formel (II)

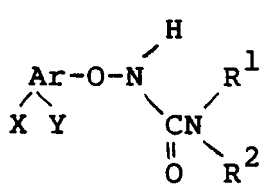

(II)

in der Ar, X, Y, $R^1$ und $R^2$ wie bei Formel (I) definiert sind,
mit einer Verbindung der folgenden Formel (III)

$R^{31}$-Cl (III)

in der $R^{31}$ wie bei Formel (I)-1 definiert ist, in Gegenwart einer Base.

**7.** Verfahren zur Herstellung eines Aryloxyharnstoffs folgender Formel (I)-2

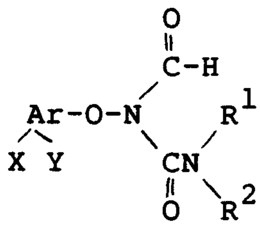

(I)-2

in der Ar, X, Y, $R^1$ und $R^2$ wie bei Formel (I) definiert sind, folgendes umfassend: das Umsetzen einer Verbindung folgender Formel (II)

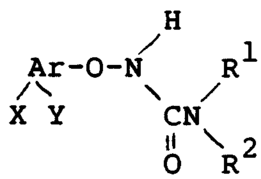

(II)

wobei Ar, X, Y, $R^1$ und $R^2$ wie bei Formel (I) definiert sind,
mit einer Verbindung der folgenden Formel (IV)

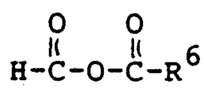

(IV)

in der $R^6$ für eine niedere Alkylgruppe steht.

**8.** Verfahren zur Herstellung eines Aryloxyharnstoffs folgender Formel (I)-3

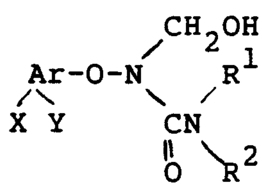

(I)-3

in der Ar, X, Y, $R^1$ und $R^2$ wie bei Formel (I) definiert sind,
folgendes umfassend: das Umsetzen einer Verbindung folgender Formel (II)

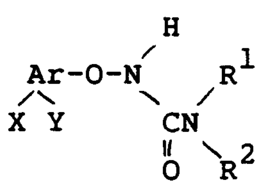

(II)

in der Ar, X, Y, $R^1$ und $R^2$ wie bei Formel (I) definiert sind,
mit Formaldehyd.

**9.** Verfahren zur Herstellung eines Aryloxyharnstoffs der folgenden Formel (I)-4

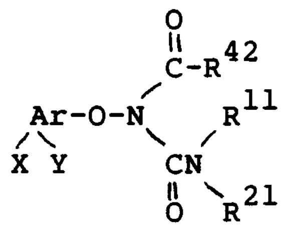

(I)-4

in der Ar, X und Y wie bei Formel (I) definiert sind, $R^{11}$ für eine niedere Alkyl-, niedere Alkenyl-, niedere Alkinyl- oder für eine $C_3$-$C_7$-Cycloalkylgruppe steht, $R^{21}$ eine niedere Alkylgruppe ist, und $R^{11}$ und $R^{21}$, zusammengefaßt, zusammen mit dem Stickstoffatom, an das sie gebunden sind, eine 4- bis 8-gliedrige heterocyclische Gruppe bilden können, wobei die heterocyclische Gruppe gegebenenfalls ein Sauerstoffatom als ein Ringbestandteil enthalten kann oder durch eine niedere Alkyl- oder Alkylengruppe substituiert sein kann, und $R^{42}$ für eine niedere Alkoxy-, nieder-alkoxysubstituierte niedere Alkoxy- oder für eine halogensubstituierte niedere Alkoxygruppe steht,
folgendes umfassend: das Umsetzen einer Verbindung der folgenden Formel (V)

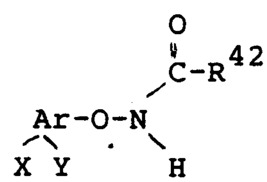

(V)

in der Ar, X und Y wie bei Formel (I) definiert sind und $R^{42}$ wie bei Formel (I)-4 definiert ist,
mit einem Carbamoylchlorid der folgenden Formel (VI)

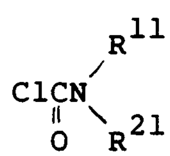

(VI)

in der $R^{11}$ und $R^{21}$ wie bei Formel (I)-4 definiert sind.

**10.** Verfahren zur Herstellung eines Aryloxyharnstoffs der folgenden Formel (I)-5

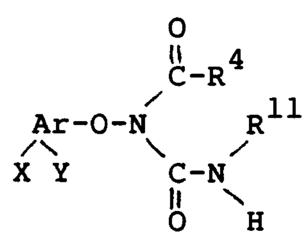

(I)-5

in der Ar, X, Y, $R^1$ und $R^4$ wie bei Formel (I) definiert sind,
folgendes umfassend: das Umsetzen einer Verbindung der folgenden Formel (VII)

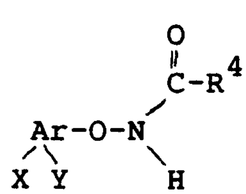

(VII)

in der Ar, X, Y und $R^4$ wie bei Formel (I) definiert sind, mit einem Isocyanatester der folgenden

Formel (VIII)

$O=C=N-R^{12}$ (VIII)

in der $R^{12}$ für eine niedere Alkyl-, niedere Alkenyl-, niedere Alkinyl- oder für eine $C_3$-$C_7$-Cycloalkylgruppe steht.

**11.** Verfahren zur Herstellung eines Aryloxyharnstoffs der folgenden Formel (I)-6

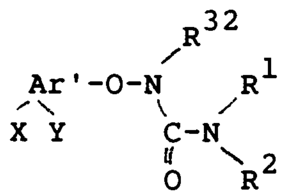

(I)-6

folgendes umfassend: das Umsetzen einer Verbindung der folgenden Formel (IX)

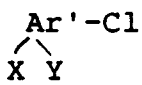

(IX)

in der Ar' eine Gruppe ist, die aus Pyrimidinyl- und Pyridazinylgruppen ausgewählt wird, und wobei $R^{32}$ für eine niedere Alkylgruppe steht,
mit einem N-Hydroxyharnstoff der folgenden Formel (X)

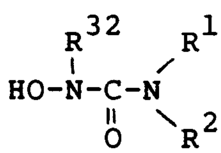

(X)

in der $R^1$ und $R^2$ wie bei Formel (I) definiert sind und $R^{32}$ wie bei Formel (X) definiert ist,
in Gegenwart einer Base.

**12.** Herbizid, welches einen Aryloxyharnstoff der Formel (I) von Anspruch 1 oder sein Säureadditionssalz als einen aktiven Bestandteil umfaßt.

**Patentansprüche für folgenden Vertragsstaat : AT**

**1.** Verfahren zur Herstellung eines Aryloxyharnstoffs der folgenden Formel (I)

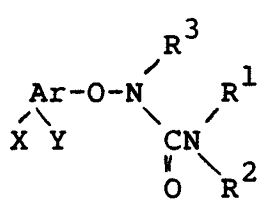

(I)

in der Ar eine Gruppe repräsentiert, welche aus der Klasse, die aus Phenyl-, Pyridyl-, Pyridazinyl-, Pyrimidinyl- und Pyrazinylgruppen besteht, ausgewählt wird,

X und Y identisch oder unterschiedlich sind und je für ein Wasserstoff- oder Halogenatom oder für eine Cyano-, Trifluormethyl-, Nitro-, niedere Alkoxy-, niedere Alkylthio- oder niedere Alkoxycarbonylgruppe stehen,

$R^1$ ein Wasserstoffatom oder eine niedere Alkyl-, niedere Alkenyl-, niedere Alkinyl- oder eine $C_3$-$C_7$-Cycloalkylgruppe bedeutet,

$R^2$ für ein Wasserstoffatom oder eine niedere Alkylgruppe steht,

$R^1$ und $R^2$, zusammengefaßt, zusammen mit dem Stickstoffatom, an das sie gebunden sind, eine 4- bis 8-gliedrige heterocyclische Gruppe bilden können, wobei die heterocyclische Gruppe ein Sauerstoffatom als ein Ringbestandteil enthalten kann oder durch eine niedere Alkylgruppe oder eine niedere Alkylengruppe substituiert sein kann,

$R^3$ für eine Gruppe der Formel $-COR^4$, eine Gruppe der Formel $-CH_2OR^5$, oder für eine niedere Alkylgruppe steht,

$R^4$ ein Wasserstoffatom oder eine niedere Alkyl-, niedere Halogenalkyl-, niedere Alkoxymethyl-, niedere Alkoxy-, nieder-alkoxysubstituierte niedere Alkoxy- oder eine halogensubstituierte niedere Alkoxygruppe bedeutet, und

$R^5$ für ein Wasserstoffatom oder eine niedere Alkylgruppe steht; und

eines Säureadditionssalzes desselben;

wobei zur Herstellung eines Aryloxyharnstoffs der folgenden Formel (I)-1

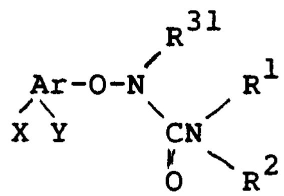

(I)-1

in der Ar, X, Y, $R^1$ und $R^2$ wie bei Formel (I) definiert sind und $R^{31}$ für eine Gruppe der Formel $-COR^{41}$, eine Gruppe der Formel $-CH_2OR^{51}$oder für eine niedere Alkylgruppe steht, $R^{41}$ eine niedere Alkyl-, niedere Halogenalkyl-, niedere Alkoxymethyl-, niedere Alkoxy-, nieder-alkoxysubstituierte niedere Alkoxy- oder eine halogensubstituierte niedere Alkoxygruppe bedeutet, und $R^{51}$ für eine niedere Alkylgruppe steht,

eine Verbindung der folgenden Formel (II)

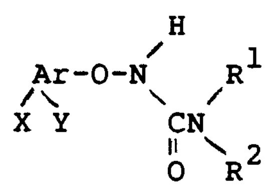

(II)

in der Ar, X, Y, $R^1$ und $R^2$ wie bei Formel (I) definiert sind,

mit einer Verbindung der folgenden Formel (III)

$R^{31}$-Cl (III)

in der $R^{31}$ wie bei Formel (I)-1 definiert ist, in Gegenwart einer Base umgesetzt wird;

oder wobei zur Herstellung eines Aryloxyharnstoffs der folgenden Formel (I)-2

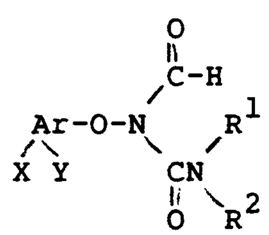

(I)-2

in der Ar, X, Y, $R^1$ und $R^2$ wie bei Formel (I) definiert sind, eine Verbindung der folgenden Formel (II)

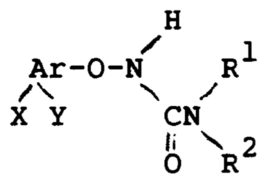

(II)

in der Ar, X, Y, $R^1$ und $R^2$ wie bei Formel (I) definiert sind, mit einer Verbindung der folgenden Formel (IV)

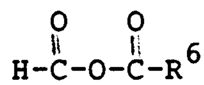

(IV)

in der $R^6$ eine niedere Alkylgruppe bedeutet, umgesetzt wird;
oder wobei zur Herstellung eines Aryloxyharnstoffs der folgenden Formel (I)-3

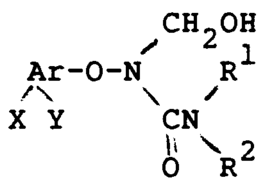

(I)-3

in der Ar, X, Y, $R^1$ und $R^2$ wie bei Formel (I) definiert sind,
eine Verbindung der folgenden Formel (II)

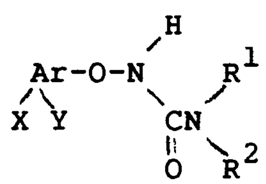

(II)

in der Ar, X, Y, $R^1$ und $R^2$ wie bei Formel (I) definiert sind,
mit Formaldehyd umgesetzt wird;
oder wobei zur Herstellung eines Aryloxyharnstoffs der folgenden Formel (I)-4

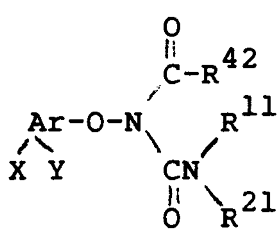

(I)-4

in der Ar, X und Y wie bei Formel (I) definiert sind, $R^{11}$ für eine niedere Alkyl-, niedere Alkenyl-, niedere Alkinyl- oder für eine $C_3$-$C_7$-Cycloalkylgruppe steht, $R^{21}$ eine niedere Alkylgruppe ist und $R^{11}$ und $R^{21}$, zusammengefaßt, zusammen mit dem Stickstoffatom, an das sie gebunden sind, eine 4- bis 8-gliedrige heterocyclische Gruppe bilden können, wobei die heterocyclische Gruppe gegebenenfalls ein Sauerstoffatom als ein Ringbestandteil enthalten kann oder durch eine niedere Alkyl- oder Alkylengruppe substituiert sein kann, und $R^{42}$ für eine niedere Alkoxy-, nieder-alkoxysubstituierte niedere Alkoxy- oder für eine halogensubstituierte niedere Alkoxygruppe steht,
eine Verbindung der folgenden Formel (V)

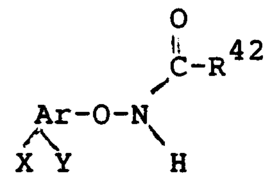

(V)

in der Ar, X und Y wie bei Formel (I) definiert sind und $R^{42}$ wie bei Formel (I)-4 definiert ist, mit einem Carbamoylchlorid der folgenden Formel (VI)

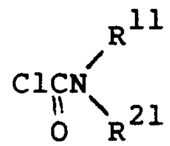

(VI)

in der $R^{11}$ und $R^{21}$ wie bei Formel (I)-4 definiert sind, umgesetzt wird;
oder wobei zur Herstellung eines Aryloxyharnstoffs, repräsentiert durch die folgende Formel (I)-5

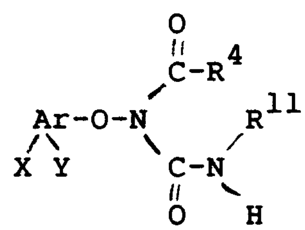

(I)-5

in der Ar, X, Y, $R^1$ und $R^4$ wie bei Formel (I) definiert sind,
eine Verbindung der folgenden Formel (VII)

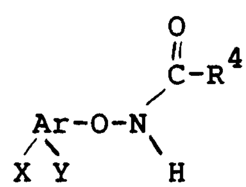

(VII)

in der Ar, X, Y und $R^4$ wie bei Formel (I) definiert sind, mit einem Isocyanatester der folgenden Formel (VIII)

$O=C=N-R^{12}$ (VIII)

in der $R^{12}$ eine niedere Alkyl-, niedere Alkenyl-, niedere Alkinyl- oder eine $C_3$-$C_7$-Cycloalkylgruppe bedeutet, umgesetzt wird;
oder wobei zur Herstellung eines Aryloxyharnstoffs der folgenden Formel (I)-6

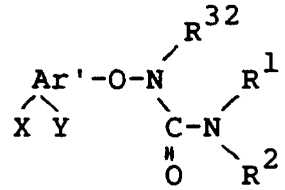

(I)-6

eine Verbindung der folgenden Formel (IX)

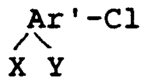

(IX)

in der Ar' eine Gruppe ist, die aus Pyrimidinyl- und Pyridazinylgruppen ausgewählt wird, und wobei $R^{32}$ für eine niedere Alkylgruppe steht,
mit einem N-Hydroxyharnstoff der folgenden Formel (X)

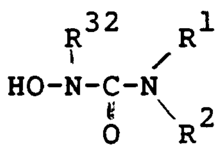

(X)

in der $R^1$ und $R^2$ wie bei Formel (I) definiert sind und $R^{32}$ wie bei Formel (X) definiert ist,
in Gegenwart einer Base umgesetzt wird.

2. Verfahren nach Anspruch 1, wobei in Formel (I) X und Y unabhängig voneinander für ein Wasserstoff- oder ein Chloratom oder für eine Trifluormethylgruppe stehen.

3. Verfahren nach Anspruch 1, wobei in Formel (I) Ar eine Phenyl- oder Pyridylgruppe ist.

4. Verfahren nach Anspruch 1, wobei in Formel (I) $R^1$ und $R^2$, zusammengefaßt, zusammen mit dem Stickstoffatom, an das sie gebunden sind, eine 6- oder 7-gliedrige heterocyclische Gruppe bilden.

5. Verfahren nach Anspruch 1, wobei in Formel (I)

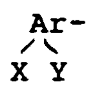

für 3,5-Dichlorphenyl oder 3-Chlor-5-trifluormethyl-2-pyridyl steht,

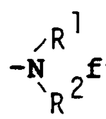 für 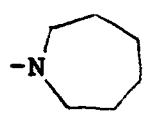

oder

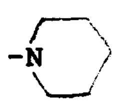

steht,
und $R^3$ für Ethoxycarbonyl, n-Propoxycarbonyl oder für Isopropoxycarbonyl steht.

6. Herbizid, welches einen Aryloxyharnstoff der Formel (I) von Anspruch 1 oder sein Säureadditionssalz als einen aktiven Bestandteil umfaßt.

**Revendications**
**Revendications pour les Etats contractants suivants : BE, CH, DE, FR, GB, IT, LI, NL**

1. Aryloxyurée représentée par la formule (I) suivante :

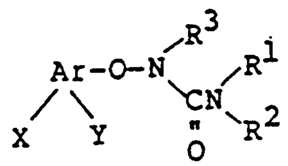 ..... (I)

dans laquelle :

- Ar représente un groupe choisi dans la classe constituée par les groupes phényle, pyridyle, pyridazinyle, pyrimidinyle et pyrazinyle ;
- X et Y sont identiques ou différents, et représentent chacun un atome d'hydrogène ou d'halogène, ou un groupe cyano, trifluorométhyle, nitro, alcoxy inférieur, alkylthio inférieur ou alcoxycarbonyle inférieur ;
- $R^1$ représente un atome d'hydrogène, ou un groupe alkyle inférieur, alcényle inférieur, alcynyle inférieur ou cycloalkyle en $C_3$-$C_7$ ;
- $R^2$ représente un atome d'hydrogène, ou un alkyle inférieur ;
- $R^1$ et $R^2$, pris ensemble, peuvent former un groupe hétérocyclique à 4 à 8 chaînons conjointement avec l'atome d'azote auquel ils sont liés, le groupe hétérocyclique pouvant contenir un atome d'oxygène en tant qu'atome de chaînon du cycle ou pouvant être substitué par un groupe alkyle inférieur ou un groupe alkylène inférieur ;
- $R^3$ représente un groupe de la formule $-COR^4$, un groupe de la formule $-CH_2OR^5$, ou un groupe alkyle inférieur ;
- $R^4$ représente un atome d'hydrogène, ou un groupe alkyle inférieur, haloalkyle inférieur, alcoxyméthyle inférieur, alcoxy inférieur, alcoxy inférieur substitué par alcoxy inférieur ou alcoxy inférieur substitué par halogène ; et
- $R^5$ représente un atome d'hydrogène ou un groupe alkyle inférieur ; et

un sel d'addition d'acide de ce composé.

**2.** Composés selon la revendication 1, dans lesquels, dans la formule (I), X et Y, indépendamment l'un de l'autre, représentent un atome d'hydrogène ou de chlore ou un groupe trifluorométhyle.

**3.** Composés selon la revendication 1, dans lesquels, dans la formule (I), Ar représente un groupe phényle ou pyridyle.

**4.** Composés selon la revendication 1, dans lesquels, dans la formule (I), $R^1$ et $R^2$, pris ensemble, forment un groupe hétérocyclique à 6 ou 7 chaînons conjointement avec l'atome d'azote auquel ils sont liés.

**5.** Composés selon la revendication 1, dans lesquels, dans la formule (I),

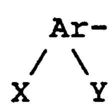

représente dichloro-3,5 phényle, ou chloro-3 trifluorométhyl-5 pyridyle-2,

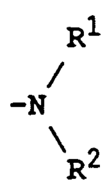

représente

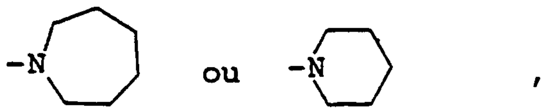

et $R^3$ représente éthoxycarbonyle, n-propoxycarbonyle ou isopropoxycarbonyle.

**6.** Procédé de fabrication d'une aryloxyurée représentée par la formule (I)-1 suivante :

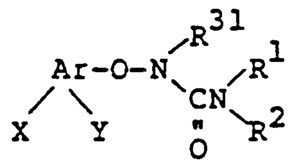

(I)-1

dans laquelle :

- Ar, X, Y, $R^1$ et $R^2$ sont tels que définis en ce qui concerne la formule (I) ; et
- $R^{31}$ représente un groupe de la formule $-COR^{41}$, un groupe de la formule $-CH_2OR^{51}$, ou un groupe alkyle inférieur,
  - $R^{41}$ représente un groupe alkyle inférieur, haloalkyle inférieur, alcoxy inférieur-méthyle, alcoxy inférieur, alcoxy inférieur substitué par alcoxy inférieur ou alcoxy inférieur substitué par halogène ; et
  - $R^{51}$ représente un groupe alkyle inférieur,

qui comprend la réaction d'un composé représenté par la formule (II) suivante :

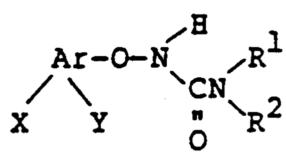

(II)

dans laquelle Ar, X, Y, $R^1$ et $R^2$ sont tels que définis en ce qui concerne la formule (I), avec un composé représenté par la formule (III) suivante :

$R^{31}$-Cl (III)

dans laquelle $R^{31}$ est tel que défini en ce qui concerne la formule (I)-1, en présence d'une base.

**7.** Procédé de fabrication d'une aryloxyurée représentée par la formule (I)-2 suivante :

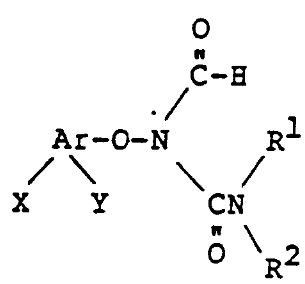

(I)-2

dans laquelle Ar, X, Y, $R^1$ et $R^2$ sont tels que définis en ce qui concerne la formule (I), qui comprend la réaction d'un composé représenté par la formule (II) suivante :

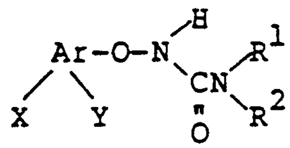

(II)

dans laquelle Ar, X, Y, $R^1$ et $R^2$ sont tels que définis en ce qui concerne la formule (I), avec un composé représenté par la formule (IV) suivante :

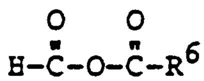

(IV)

dans laquelle $R^6$ représente un groupe alkyle inférieur.

**8.** Procédé de fabrication d'une aryloxyurée représentée par la formule (I)-3 suivante :

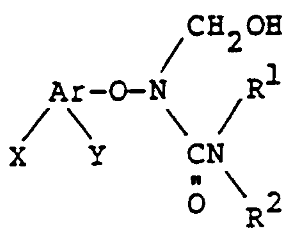

(I)-3

dans laquelle Ar, X, Y, $R^1$ et $R^2$ sont tels que définis en ce qui concerne la formule (I), qui comprend la réaction d'un composé représenté par la formule (II) suivante :

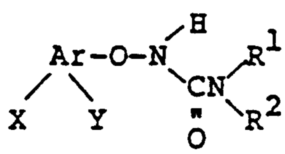

(II)

dans laquelle Ar, X, Y, $R^1$ et $R^2$ sont tels que définis en ce qui concerne la formule (I), avec le formaldéhyde.

**9.** Procédé de fabrication d'une aryloxyurée représentée par la formule (I)-4 suivante :

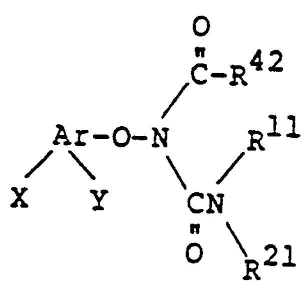

(I)-4

dans laquelle :

- Ar, X et Y sont tels que définis en ce qui concerne la formule (I) ;
- $R^{11}$ représente un groupe alkyle inférieur, alcényle inférieur, alcynyle inférieur ou cycloalkyle en $C_3$-$C_7$ ;
- $R^{21}$ représente un groupe alkyle inférieur ; et $R^{11}$ et $R^{21}$, pris ensemble, peuvent former un groupe

hétérocyclique à 4 à 8 chaînons conjointement avec l'atome d'azote auquel ils sont liés, ledit groupe hétérocyclique contenant de façon facultative un atome d'oxygène en tant qu'atome de chaînon du cycle ou étant substitué par un groupe alkyle ou alkylène inférieur ; et

- $R^{42}$ représente un groupe alcoxy inférieur, alcoxy inférieur substitué par alcoxy inférieur, ou alcoxy inférieur substitué par halogène,

qui comprend la réaction d'un composé représenté par la formule (V) suivante :

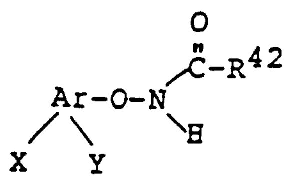

(V)

dans laquelle :

- Ar, X et Y, sont tels que définis en ce qui concerne la formule (I) ; et
- $R^{42}$ est tel que défini en ce qui concerne la formule (I)-4,

avec un chlorure de carbamoyle représenté par la formule (VI) suivante :

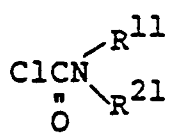

(VI)

dans laquelle $R^{11}$ et $R^{21}$ sont tels que définis en ce qui concerne la formule (I)-4.

**10.** Procédé de fabrication d'une aryloxyurée représentée par la formule (I)-5 suivante :

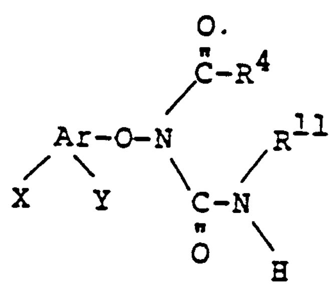

(I)-5

dans laquelle Ar, X, Y, $R^1$ et $R^4$ sont tels que définis ci-dessus en ce qui concerne la formule (I), qui comprend la réaction d'un composé représenté par la formule (VII) suivante :

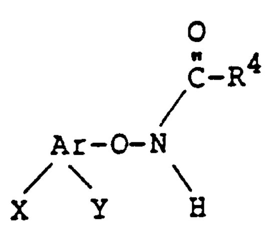

(VII)

dans laquelle Ar, X, Y et $R^4$ sont tels que définis ci-dessus en ce qui concerne la formule (I), avec un ester isocyanate représenté par la formule (VIII) suivante :

$O=C=N\text{-}R^{12}$ (VIII)

dans laquelle $R^{12}$ représente un groupe alkyle inférieur, alcényle inférieur, alcynyle inférieur ou cycloalkyle en $C_3$-$C_7$.

**11.** Procédé de fabrication d'une aryloxyurée représentée par la formule (I)-6 suivante :

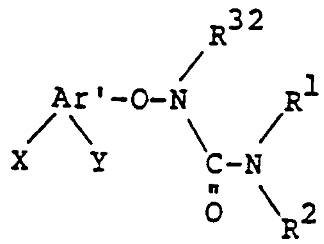

(I)-6

qui comprend la réaction d'un composé représenté par la formule (IX) suivante :

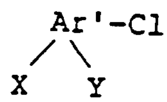

(IX)

dans laquelle :

- Ar' représente un groupe choisi parmi les groupes pyrimidinyle et pyridazinyle ; et
- $R^{32}$ représente un groupe alkyle inférieur,

avec une N-hydroxyurée représentée par la formule (X) suivante :

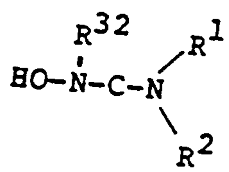

(X)

dans laquelle :

- $R^1$ et $R^2$ sont tels que définis en ce qui concerne la formule (I) ; et
- $R^{32}$ est tel que défini en ce qui concerne la formule (X),

en présence d'une base.

**12.** Herbicide comprenant une aryloxyurée représentée par la formule (I) telle que définie à la revendication 1, ou son sel d'addition d'acide, en tant qu'ingrédient actif.

**Revendications pour l'Etat contractant suivant : AT**

**1.** Procédé de fabrication d'une aryloxyurée représentée par la formule (I) suivante :

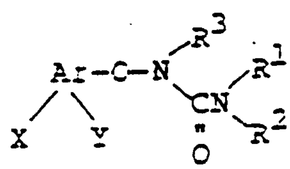

..... (I)

dans laquelle :

- Ar représente un groupe choisi dans la classe constituée par les groupes phényle, pyridyle, pyridazinyle, pyrimidinyle et pyrazinyle ;
- X et Y sont identiques ou différents, et représentent chacun un atome d'hydrogène ou d'halogène, ou un groupe cyano, trifluorométhyle, nitro, alcoxy inférieur, alkylthio inférieur ou alcoxycarbonyle inférieur ;
- $R^1$ représente un atome d'hydrogène, ou un groupe alkyle inférieur, alcényle inférieur, alcynyle inférieur ou cycloalkyle en $C_3$-$C_7$ ;

- $R^2$ représente un atome d'hydrogène, ou un alkyle inférieur ;
- $R^1$ et $R^2$, pris ensemble, peuvent former un groupe hétérocyclique à 4 à 8 chaînons conjointement avec l'atome d'azote auquel ils sont liés, le groupe hétérocyclique pouvant contenir un atome d'oxygène en tant qu'atome de chaînon du cycle ou pouvant être substitué par un groupe alkyle inférieur ou un groupe alkylène inférieur ;
- $R^3$ représente un groupe de la formule $-COR^4$, un groupe de la formule $-CH_2OR^5$, ou un groupe alkyle inférieur ;
- $R^4$ représente un atome d'hydrogène, ou un groupe alkyle inférieur, haloalkyle inférieur, alcoxyméthyle inférieur, alcoxy inférieur, alcoxy inférieur substitué par alcoxy inférieur ou alcoxy inférieur substitué par halogène ; et
- $R^5$ représente un atome d'hydrogène ou un groupe alkyle inférieur ; et

d'un sel d'addition d'acide de ce composé ;

- dans lequel, pour la fabrication d'une aryloxyurée représenté par la formule (I)-1 suivante :

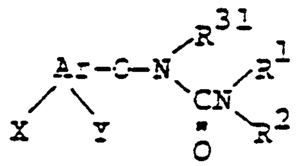

(I)-1

dans laquelle :

- Ar, X, Y, $R^1$ et $R^2$ sont tels que définis en ce qui concerne la formule (I) ; et
- $R^{31}$ représente un groupe de la formule $-COR^{41}$, un groupe de la formule $-CH_2OR^{51}$, ou un groupe alkyle inférieur,
  - $R^{41}$ représente un groupe alkyle inférieur, haloalkyle inférieur, alcoxy inférieurméthyle, alcoxy inférieur, alcoxy inférieur substitué par alcoxy inférieur ou alcoxy inférieur substitué par halogène ; et
  - $R^{51}$ représente un groupe alkyle inférieur,

on fait réagir un composé représenté par la formule (II) suivante :

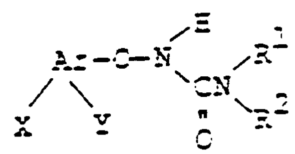

(II)

dans laquelle Ar, X, Y, $R^1$ et $R^2$ sont tels que définis en ce qui concerne la formule (I), avec un composé représenté par la formule (III) suivante :

$R^{31}$-Cl (III)

dans laquelle $R^{31}$ est tel que défini en ce qui concerne la formule (I)-1, en présence d'une base ;

- ou dans lequel, pour la fabrication d'une aryloxyurée représentée par la formule (I)-2 suivante :

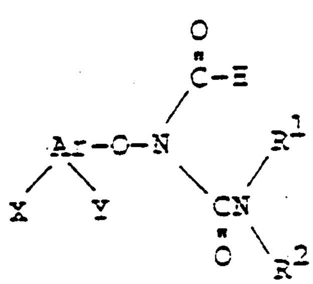

(I)-2

dans laquelle Ar, X, Y, $R^1$ et $R^2$ sont tels que définis en ce qui concerne la formule (I),

on fait réagir un composé représenté par la formule (II) suivante :

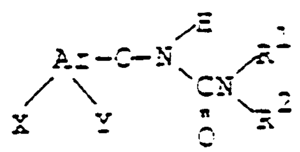

(II)

dans laquelle Ar, X, Y, $R^1$ et $R^2$ sont tels que définis en ce qui concerne la formule (I), avec un composé représenté par la formule (IV) suivante :

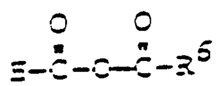

(IV)

dans laquelle $R^6$ représente un groupe alkyle inférieur ;

- ou dans lequel, pour la fabrication d'une aryloxyurée représentée par la formule (I)-3 suivante :

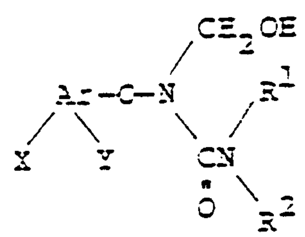

(I)-3

dans laquelle Ar, X, Y, $R^1$ et $R^2$ sont tels que définis en ce qui concerne la formule (I), on fait réagir un composé représenté par la formule (II) suivante :

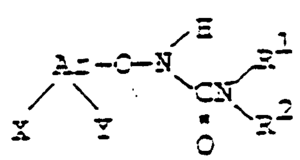

(II)

dans laquelle Ar, X, Y, $R^1$ et $R^2$ sont tels que définis en ce qui concerne la formule (I), avec le formaldéhyde ;

- ou dans lequel, pour la fabrication d'une aryloxyurée représentée par la formule (I)-4 suivante :

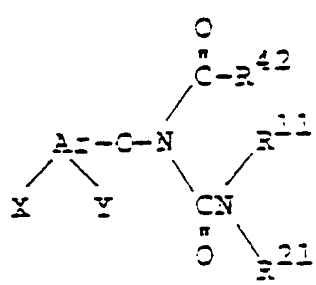

(I)-4

dans laquelle :

- Ar, X et Y sont tels que définis en ce qui concerne la formule (I) ;
- $R^{11}$ représente un groupe alkyle inférieur, alcényle inférieur, alcynyle inférieur ou cycloalkyle en $C_3$-$C_7$ ;
- $R^{21}$ représente un groupe alkyle inférieur ; et

- $R^{11}$ et $R^{21}$, pris ensemble, peuvent former un groupe hétérocyclique à 4 à 8 chaînons conjointement avec l'atome d'azote auquel ils sont liés, ledit groupe hétérocyclique contenant de façon facultative un atome d'oxygène en tant qu'atome de chaînon du cycle ou étant substitué par un groupe alkyle ou alkylène inférieur ; et
- $R^{42}$ représente un groupe alcoxy inférieur, alcoxy inférieur substitué par alcoxy inférieur, ou alcoxy inférieur substitué par halogène,

on fait réagir un composé représenté par la formule (V) suivante :

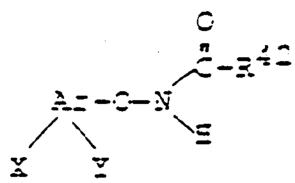

(V)

dans laquelle :
- Ar, X et Y, sont tels que définis en ce qui concerne la formule (I) ; et
- $R^{42}$ est tel que défini en ce qui concerne la formule (I)-4,

avec un chlorure de carbamoyle représenté par la formule (VI) suivante :

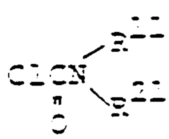

(VI)

dans laquelle $R^{11}$ et $R^{21}$ sont tels que définis en ce qui concerne la formule (I)-4 ;

- ou dans lequel, pour la fabrication d'une aryloxyurée représentée par la formule (I)-5 suivante :

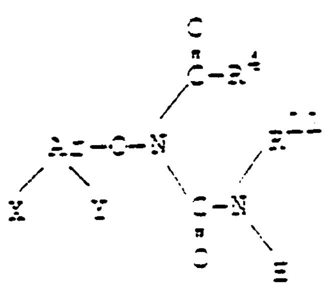

(I)-5

dans laquelle Ar, X, Y, $R^1$ et $R^4$ sont tels que définis ci-dessus en ce qui concerne la formule (I), on fait réagir un composé représenté par la formule (VII) suivante :

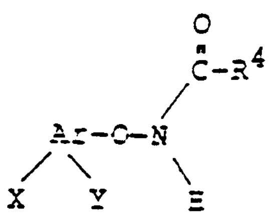

(VII)

dans laquelle Ar, X, Y et $R^4$ sont tels que définis ci-dessus en ce qui concerne la formule (I), avec un ester isocyanate représenté par la formule (VIII) suivante :

$O=C=N-R^{12}$ (VIII)

dans laquelle $R^{12}$ représente un groupe alkyle inférieur, alcényle inférieur, alcynyle inférieur ou cycloalkyle en $C_3$-$C_7$ ;

- ou dans lequel, pour la fabrication d'une aryloxyurée représentée par la formule (I)-6 suivante :

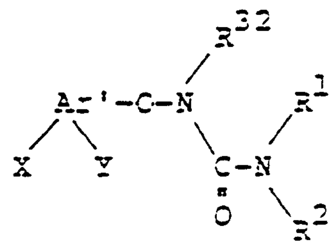

(I)-6

on fait réagir un composé représenté par la formule (IX) suivante :

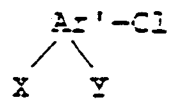

(IX)

dans laquelle :
- Ar' représente un groupe choisi parmi les groupes pyrimidinyle et pyridazinyle ; et
- $R^{32}$ représente un groupe alkyle inférieur,

avec une N-hydroxyurée représentée par la formule (X) suivante :

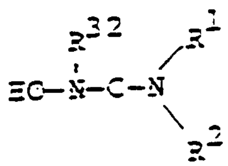

(X)

dans laquelle :
- $R^1$ et $R^2$ sont tels que définis en ce qui concerne la formule (I) ; et
- $R^{32}$ est tel que défini en ce qui concerne la formule (X),

en présence d'une base.

2. Procédé selon la revendication 1, dans lequel, dans la formule (I), X et Y, indépendamment l'un de l'autre, représentent un atome d'hydrogène ou de chlore ou un groupe trifluorométhyle.

3. Procédé selon la revendication 1, dans lequel, dans la formule (I), Ar représente un groupe phényle ou pyridyle.

4. Procédé selon la revendication 1, dans lequel, dans la formule (I), $R^1$ et $R^2$, pris ensemble, forment un groupe hétérocyclique à 6 ou 7 chaînons conjointement avec l'atome d'azote auquel ils sont liés.

5. Procédé selon la revendication 1, dans lequel, dans la formule (I),

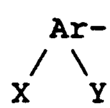

représente dichloro-3,5 phényle, ou chloro-3 trifluorométhyl-5 pyridyle-2,

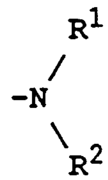

représente

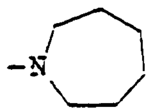 ou 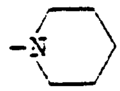 ,

et $R^3$ représente éthoxycarbonyle, n-propoxycarbonyle ou isopropoxycarbonyle.

**6.** Herbicide comprenant une aryloxyurée représentée par la formule (I) telle que définie à la revendication 1, ou son sel d'addition d'acide, en tant qu'ingrédient actif.